# EUROPEAN PATENT SPECIFICATION

(11) **EP 4 353 836 B1**
(45) Date of publication and mention of the grant of the patent: **20.08.2025**
(21) Application number: 24160473.5
(22) Date of filing: 26.04.2019
(51) Int. Cl.: C12Q 1/6869

(54) **HIGHLY ACCURATE SEQUENCING METHOD**
HOCHGENAUES SEQUENZIERUNGSVERFAHREN
PROCÉDÉ DE SÉQUENÇAGE HAUTEMENT PRÉCIS

(30) Priority: 27.04.2018 JP 2018086972; 07.09.2018 JP 2018168288
(43) Date of publication of application: 17.04.2024
(62) Divisional of application: 19724948.5
(73) Proprietor: Kao Corporation, Tokyo 103-8210 (JP)
(72) Inventor: MATSUMURA, Shoji, Tochigi, 321-3497 (JP); SATO, Hirayuki, Tochigi, 321-3497 (JP); OTSUBO, Yuki, Tochigi, 321-3497 (JP)
(74) Representative: Vossius & Partner Patentanwälte Rechtsanwälte mbB

(56) References cited:
- WO-A1-2013/181170
- WO-A1-2016/109452
- WO-A1-2018/035170
- RALEY CASTLE ET AL: "Preparation of next-generation DNA sequencing libraries from ultra-low amounts of input DNA: Application to single-molecule, real-time (SMRT) sequencing on the Pacific Biosciences RS II.", BIORXIV, 25 March 2014 (2014-03-25), XP055982808, Retrieved from the Internet <URL:https://www.biorxiv.org/content/10.1101/003566v1.full.pdf> [retrieved on 20221117], DOI: 10.1101/003566

## Description

### Field

The present invention as defined in the appended claims provides a method for sequencing DNA. A mutation analysis method using the method for sequencing DNA is described.

### Background

The technology of next-generation sequencing (NGS) has significantly developed and has been widely used for sequencing genomic DNA or the like in recent years (Non Patent Literature 1). In genomic mutation analysis for cancer cells, for example, NGS has enabled not only analysis focusing on a specific gene region but also mutation analysis for the whole genome, which used to be difficult, producing various new findings (Non Patent Literatures 2, 3).

Various sequencers have previously been developed, and representative examples thereof include the sequencers HiSeq and MiSeq from Illumina, Inc. DNA from a cell, tissue, or the like of a target of analysis is finely fragmented into a size of several hundreds of bp, and these sequencers acquire a large amount of sequence information (read sequences) on the fragmented sequences concurrently and in parallel to use the sequence information for data analysis. Although the reading accuracy for each individual read sequence is not very high, this system allows to collect read sequences derived from a plurality of cells and acquire information on bases appearing with a high frequency among the read sequences. Such an analysis corresponds to a plurality of sequencing on the same part in the genome sequence and achieves highly accurate sequencing. The sequencing data acquired can be used for mutation analysis such as identification of single nucleotide polymorphism in a human or gene mutation in cancer cells.

Mutations detectable with the above sequencing technique are those commonly included in a relatively large number of cells in a target cell population, such as single nucleotide polymorphism and mutations in cancer cells. The error frequency of NGS for each individual read sequence is said to be approximately 1/10³ bp, and hence it is generally difficult for the above sequencing technique to accurately identify mutations with lower frequency (e.g. 0.1% or lower). Examples of cases with such lower frequency mutations include rare mutations only possessed by a few cell populations in a cancer tissue, and mutations independently generated in individual cells in a target cell population through exposure to environmental mutagens or the like. Currently, there are few examples using NGS for analysis of low-frequency mutations. If large-scale identification of low-frequency mutations with NGS is successfully realized, however, NGS is expected to be applicable to early detection of cancer and to a method for toxicity evaluation for mutagens such as chemicals. In addition, comprehensive analysis of mutations possessed by individual cells in a human can be expected to be useful for various applications such as prevention of cancer by estimating environmental mutagens to which a human subject has been previously exposed.

Low-frequency somatic cell mutations have been reported to be identifiable by analyzing an isolated single cell. For example, Gundry et al. have identified a mutation due to a chemical by whole-genome sequencing for a single cell from cells of a mouse or the like exposed to a mutagen (Non Patent Literature 4). However, this technique is disadvantageous, for example, in that it may be difficult to apply to cancer tissues whose isolation culture is difficult, that isolation of a cell is time-consuming, and that the outcome is only mutation information derived from a single cell.

Hence, a sequencing method has been reported which accurately identifies rare mutations present in a cell population through enhanced sequencing accuracy for individual DNA fragments, without involving isolation of a single cell. Examples of such methods widely used include a method of overlapping each read pair acquired in paired-end sequencing with a sequencer from Illumina, Inc. (Non Patent Literature 5). This is a method capable of enhancing sequencing accuracy by building consensus between a read pair acquired for each fragment.

However, errors in NGS include those due to oxidative modification caused to target DNA during library preparation or storage, or the like (Non Patent Literatures 6, 7). It is difficult to remove such errors in the above-mentioned method of overlapping each read pair. Hence, a method has been developed which distinguish between errors and true mutations by using sequencing information on two complementary strands of DNA (Non Patent Literatures 8 to 10, Patent Literature 1). Because oxidative modification of DNA generally occurs only in one of two strands of DNA, use of sequencing information on two complementary strands of DNA enables selective identification of true mutations fixed in the two strands. In a Duplex Sequencing method (Non Patent Literature 8, Patent Literature 1), for example, to each DNA fragment a 12-bp tag sequence specific to the fragment is added; the DNA fragments are subjected to PCR amplification and sequencing; after the sequencing, read sequences derived from two complementary strands constituting identical DNA fragment are collected by referring to the tag sequence; and then consensus between the complementary strands is built to enhance sequencing accuracy. In addition, an SMRTbell Template method (Non Patent Literature 10) with a sequencer from Pacific Biosciences of California, Inc. can enhance sequencing accuracy by using information on complementary strands, similarly.

However, the above-described existing methods utilizing information on complementary strands need a special method for library preparation, such as addition of a tag sequence specific to a DNA fragment in library preparation, or are implementable only with specific sequencers as in the SMRTbell Template method. Accordingly, it is hard to say that the above-described methods are recognized as methods widely and generally implementable. On the other hand, a method without need of adding a tag sequence specific to a DNA fragment as described has been disclosed, only in theory, in which a partial sequence of each DNA fragment itself is used as an index to collect sequences derived from the same DNA region (Patent Literature 2). This approach is expected to be superior in versatility because it allows simplified procedures of library preparation and does not depend on the type of a sequencer. However, a risk has been pointed out for the approach that if a tag sequence specific to a DNA fragment is not added, sequence information derived from different DNA fragments may be misrecognized as information on the same fragment (Patent Literature 1). Furthermore, WO 2016/109452 A1 describes a method for the detection of genetic variants in tumor cells based on the identification of somatic mutations in polynucleotides collected from cancer cells. Samples are prepared from circulating cell-free DNA, with a size smaller than 500bp. It is described that the extracted DNA can be tagged with 2 different octomers with overloaded hairpin adaptors. For the correction of sequencing errors, the use of distinctive tags is mentioned.
(Patent Literature 1) WO2013/142389
(Patent Literature 2) WO2012/142213
(Patent Literature 3) WO2016/109452 A1
(Non Patent Literature 1) Schendure & Ji, Nature Biotechnology, 26(10):1135-1145, 2008
(Non Patent Literature 2) Stratton, Science, 331:1553-1558, 2011
(Non Patent Literature 3) Alexandrov et al. Cell Reports, 3:246-259, 2013
(Non Patent Literature 4) Gundry et al. Nucleic Acid Research, 40(5):2032-2040, 2012
(Non Patent Literature 5) Zhang et al. Bioinformatics, 30(5):614-620, 2014
(Non Patent Literature 6) Costello et al. Nucleic Acid Research, 41(6):e67, 2013
(Non Patent Literature 7) Shibutani et al. Nature, 349:431-434, 1991
(Non Patent Literature 8) Schmitt et al. PNAS, 109(36):14508-14513, 2012
(Non Patent Literature 9) Gregory et al. Nucleic Acid Research, 44(3):e22, 2016
(Non Patent Literature 10) Travers et al. Nucleic Acid Research, 38(15):e159, 2010

### Summary of the Invention

The present invention provides a method for sequencing DNA, the method comprising:
(1) preparing fragments of sample DNA;
(2) subjecting the fragments of the sample DNA to PCR to produce two or more amplified fragments for each of the fragments of the sample DNA, and obtaining PC products containing a plurality of amplified fragments,
   wherein the amount of input DNA in the PCR has been adjusted to 0.002 to 1.7 amol per 1 Mbp in size of the sample DNA;
(3) sequencing the PCR products to generate one or more read sequences from the plurality of amplified fragments, and acquiring a plurality of read sequences for the plurality of amplified fragments;
(4) collecting, from the plurality of read sequences acquired, read sequences containing sequence information on the same region in the sample DNA into a group to generate one or more groups of read sequences; and
(5) building consensus of sequence information among read sequences included in each of the groups of read sequences.

### Brief Description of Drawings

[fig.1]Figure 1 shows distribution of the number of read pairs per group for an estimated fragment in 10 Gbp sequencing data (control group) for libraries with different amounts of input DNA in PCR, where the horizontal axis represents the number of read pairs per group, the vertical axis represents the number of groups with each number of read pairs, and curves represent data for libraries with different amounts of input DNA.
[fig.2]Figure 2 shows distribution of the number of read pairs per group for an estimated fragment in 10 Gbp sequencing data (ENU group) for libraries with different amounts of input DNA in PCR, where the horizontal axis represents the number of read pairs per group, the vertical axis represents the number of groups with each number of read pairs, and curves represent data for libraries with different amounts of input DNA.
[fig.3]Figure 3 shows data efficiency for 10 Gbp sequencing data for libraries with different amounts of input DNA in PCR, where A: control group, B: ENU group.
[fig.4]Figure 4 shows distribution of the number of read pairs per group for an estimated fragment in 2 Gbp sequencing data (control group) for libraries with different amounts of input DNA in PCR, where the horizontal axis represents the number of read pairs per group, the vertical axis represents the number of groups with each number of read pairs, and curves represent data for libraries with different amounts of input DNA.
[fig.5]Figure 5 shows data efficiency for 2 Gbp sequencing data for libraries with different amounts of input DNA in PCR.
[fig.6]Figure 6 shows overlap rates for 10 Gbp sequencing data for libraries with different amounts of input DNA in PCR, where A: control group, B: ENU group.
[fig.7]Figure 7 shows genome mutation frequencies for the control group and the ENU group calculated from 10 Gbp sequencing data for libraries with different amounts of input DNA in PCR (20,000 to 156 amol).
[fig.8]Figure 8 shows genome mutation frequencies for the control group and the ENU group calculated from 10 Gbp sequencing data for libraries with different amounts of input DNA in PCR (156 to 5 amol).
[fig.9]Figure 9 shows mutation frequencies for different base pair mutation patterns in 1,000 bp synthesized DNA in which a base-pair substitution mutation has been introduced to an AT base pair.
[fig.10]Figure 10 shows the variation of overlap rates due to difference in the amount of input DNA in PCR, where the horizontal axis represents amounts of input DNA in PCR, where Control: control sample, Mutation: mutated sample.
[fig.11]Figure 11 shows efficiency of generating consensus read sequences from 10 Gbp sequencing data (control group) in Example 2, where the horizontal axis represents the number of read pairs per group, and the vertical axis represents the ratio of the number of consensus read sequences to the total number of read pairs.
[fig.12] Figure 12 shows distribution of the number of read pairs per group for an estimated fragment for Library No. 1 in Example 5, where curves represent data for different mouse chromosomes, the horizontal axis represents the number of read pair per group, and the vertical axis represents the number of groups with each number of read pairs.
[fig.13]Figure 13 shows frequencies of base-pair substitution mutations in mouse DNA.

### Detailed Description of Invention

### (1. Definitions)

"Mutation" herein refers to mutation generated in DNA, and examples thereof include deletion, insertion, substitution, addition, inversion, and translocation of a nucleotide or sequence in DNA. The mutation as used herein encompasses deletion, insertion, substitution, and addition of one nucleotide, and deletion, insertion, substitution, addition, inversion, and translocation of a sequence consisting of two or more nucleotides. The mutation as used herein also includes mutation in a gene-coding region and noncoding region, and also includes mutation involving change of an amino acid to be expressed and mutation without involving it (silent mutation).

"Genotoxicity" of substance to be evaluated in the present invention refers to a property of the substance to cause mutation (what is called mutagenicity).

A "reference sequence" herein refers to a known sequence included in DNA as a target of analysis. The known sequence to be used is preferably a sequence registered in a public database or the like, a sequence in target DNA which has been sequenced in advance by using a sequencer or the like may also be used as the known sequence. The region, length and number of the reference sequence are not limited, and can be appropriately selected from DNA in accordance with the purpose of analysis.

An "amplified fragment", which is to be obtained in PCR, herein refers to a double-stranded DNA fragment obtained in PCR amplification of template DNA.

"Two complementary strands" regarding DNA or a fragment thereof herein refer to two single strands constituting double-stranded DNA or a fragment thereof and being complementary to each other.

A "raw read sequence" herein refers to sequence information read out through sequencing of a base sequence. A "read sequence" herein refers to information on a nucleotide sequence targeted in sequencing, which is extracted from the raw read sequence by trimming adapter sequences added for PCR or sequencing reaction or bases of low quality from the raw read sequence. Nevertheless, a raw read sequence can be directly used as a read sequence if the trimming or the like is not needed. In the case that a raw read sequence contains a plurality of pieces of sequence information on base sequences targeted in sequencing, the individual sequence information on the nucleotide sequences targeted in sequencing can be extracted as individual read sequences, respectively, and in this case one or more read sequences can be generated from one raw read sequence. Accordingly, even in the case that an adapter sequence or the like is added to fragments of sample DNA, each read sequence herein fundamentally contains only information on a nucleotide sequence derived from a fragment of sample DNA, without containing sequence information on the adapter sequence or the like. Read sequences each contain information on a sequence starting from a base at one of the terminals of a nucleotide sequence of a target of sequencing (e.g. the nucleotide sequence of a fragment of sample DNA). The length of a read sequence typically depends on the performance and specification of a sequencer. Thus, each read sequence may optionally, but not necessarily need to, contain information on a sequence from a base at one terminal to a base at the other terminal (full sequence) of a nucleotide sequence of a target of sequencing.

The "beginning" and "end" of a read sequence herein refer to a terminal initially read out and terminal finally read out, respectively, in generating the read sequence. "Sequence direction" regarding a read sequence herein refers to the direction from the beginning of the read sequence to the end thereof in a DNA sequence on which the read sequence is mapped.

The situation that two or more read sequences "contain sequence information on the same region in sample DNA" herein refers to a situation that estimated positions of two terminals of the read sequences are identical in the sequence of sample DNA (or a reference sequence). The situation that two or more read sequences "contain sequence information on the same region in sample DNA" does not require a situation that the two or more read sequences are of 100% sequence identity. However, read sequences having different estimated positions of both terminals, even by 1 bp, do not "contain sequence information on the same region in sample DNA".

The situation that two or more read sequences are "mapped on the same position in a reference sequence" herein refers to a situation that on being mapped on a reference sequence, the read sequences have identical positions of two terminals in the reference sequence (wherein their directions are not limited).

A "read pair" herein refers to a pair of two read sequences read out of one sequence of a target of sequencing. One of the two read sequences included in a read pair is a read sequence containing sequence information on reading out of the sequence of a target from the 5'-terminal side to the 3'-terminal side (herein, referred to as "read 1"), and the other is a read sequence containing sequence information on reading out of the sequence of the same one strand from the 3'-terminal side to the 5'-terminal side (herein, referred to as "read 2").

A "region between the beginning of read 1 and the beginning of read 2" in DNA, a sequence, or a fragment herein refers to a region from a site at which the beginning of read 1 is positioned to a site at which the beginning of read 2 is positioned (including the site at which the beginning of read 1 is positioned and the site at which the beginning of read 2 is positioned) in the DNA, sequence, or fragment on which read 1 and read 2 are mapped.

The situation that two or more read pairs "contain sequence information on the same region in sample DNA" herein refers to a situation that a "region between the beginning of read 1 and the beginning of read 2" in the sequence of sample DNA (or a reference sequence) is the same among the read pairs. When two or more read pairs "contain sequence information on the same region in sample DNA", the read sequences of the read pairs are not necessarily required to be of 100% sequence identity. However, read pairs having different terminal position of the "region between the beginning of read 1 and the beginning of read 2", even by 1 bp, do not those "containing sequence information on the same region in sample DNA".

### (2. Method for sequencing DNA)

In applying a highly accurate sequencing method to a method for toxicity evaluation for chemicals or mutation analysis for a certain individual, simplification of the procedure is effective. The present invention as defined in the claims provides a sequencing method capable of achieving high reading accuracy in a simpler manner. Further, the present disclosure provides the optimum conditions and applicable conditions for the sequencing method.

The method according to the present invention as defined in the claims allows to collect sequence information derived from identical DNA fragments and sequence information on complementary strands forming the fragments through library preparation and experimental operations almost equivalent to the procedure of sequencing that have generally been performed in the art, and thereby allowing to conduct sequencing using the sequence information thus collected. The method does not require labeling of each individual DNA fragment with a tag sequence specific thereto, as in conventional sequencing methods, to collect sequence information on the same DNA fragment and complementary strands. This method is superior in technical simplicity and versatility to conventional methods.

The present invention as defined in the claims provides a highly accurate method for sequencing DNA. Fundamentally, this method for sequencing DNA comprises: obtaining fragments of sample DNA; subjecting the fragments of the sample DNA to PCR to obtain PCR products; sequencing the PCR products thus obtained to generate one or more reading results (read sequences) for each of amplified fragments, which are derived from one fragment of the sample DNA, contained in the PCR products, thereby acquiring a plurality of read sequences for the plurality of amplified fragments; collecting, from the read sequences acquired through the sequencing, read sequences containing sequence information on the same region in the sample DNA; and constructing sequence information on the sample DNA by using information on the collected read sequences.

### (2-1) Preparation of fragments of sample DNA and PCR

"Sample DNA" to be used in the method for sequencing DNA according to the present invention as defined in the claims is only required to be double-stranded DNA, and examples of the origin thereof include animals, plants, and microorganisms. Examples of types of the sample DNA include genomic DNA, mitochondrial genome DNA, chloroplast genome DNA, plasmid DNA, viral genome DNA, and synthesized DNA. Genomic DNA is preferred. Such sample DNA can be obtained through extraction or isolation from cells using a common method in the art. For the extraction or isolation, for example, commercially available DNA extraction kits can be used. Alternatively, DNA which has been stored after being extracted or isolated from cells may be obtained and used for the method according to the present invention. Synthesized DNA can be obtained through synthesis using any known chemical synthesis method.

Alternatively, double-stranded RNA may be used in place of double-stranded DNA in the method described. Double-stranded RNA can be extracted or isolated from viruses or cells possessing it by using a common method in the art such as commercially available RNA extraction kits. Alternatively, double-stranded RNA which has been stored after being extracted or isolated may be obtained and used for the method described. In obtaining and analyzing RNA in the method described, the RNA obtained is converted into cDNA before PCR, and bases of T in a read sequence derived from the cDNA are read as bases of U.

DNA fragments can be prepared by using a common method in the art allowing cutting at random positions, such as sonication and enzymatic treatment. Specific examples of fragmentation of DNA include intensive sonication by using DNA Shearing system from Covaris Inc. and a transposon and transposase treatment by using Nextera technology from Illumina, Inc. The length of each fragment to be prepared can be appropriately selected in accordance with lengths accurately readable for the sequencer. Although 100 to 10,000 bp can be typically selected, fragments of 10,000 bp or more in length may be prepared if the sequencer can accurately read such fragments, and a more appropriate range can be selected in accordance with the type of the sequencer. In using a sequencer for sequencing reaction involving amplification of fragments, the mean length of fragments is preferably 100 to 1,000 bp, and more preferably 200 to 500 bp. Alternatively, longer fragments may be prepared and subjected to PCR described later to prepare PCR products with appropriate lengths for sequencing reaction.

In the method according to the present invention as defined in the claims, fragments of sample DNA are subjected to PCR to obtain PCR products. The PCR can be performed in accordance with a conventional technique using commercially available reagents and apparatuses for PCR. Alternatively, a sequencer equipped with a PCR amplifier may be used. Such high-throughput sequencers involving PCR amplification of sample DNA fragments in their procedure are available on the market as e.g., HiSeq (produced by Illumina, Inc.) and MiSeq (produced by Illumina, Inc.). In the PCR, two or more amplified fragments are produced for each of the fragments of the sample DNA. Here, the two or more amplified fragments may be prepared for each of at least some of the fragments of the sample DNA used as templates for the PCR. The two or more amplified fragments may be obtained for all of the fragments of the sample DNA used as templates in the PCR, however, which is not required. Using a certain amount of PCR products is recommended in sequencing reaction with high-throughput sequencers involving PCR amplification of sample DNA fragments in their procedure, from the viewpoint of sequencing efficiency. Accordingly, it is preferred to set the amount of PCR products to the recommended level by changing the number of PCR cycles in accordance with the amount of the sample DNA to be subjected to PCR (amount of input DNA in PCR).

### (2-2) Sequencing and generation of read sequences

Subsequently, the resulting PCR products of the fragments of the sample DNA are subjected to sequencing. It is sufficient to conduct the sequencing of the PCR products for a region required for analysis or the like, e.g., for a region to be used for sequence comparison with a reference sequence in the case of mutation analysis described later. For example, it is sufficient to sequence fragments having a region at least partly, preferably totally corresponding to a DNA region of reference sequence. In the case of using mammalian cells or the like, sequencing may be selectively performed for exonic regions. For selection of a region, kits such as a SureSelect (produced by Agilent Technologies, Inc.) are available on the market.

Through the sequencing, raw read sequences for amplified fragments are acquired. Adapter sequences added for PCR or sequencing reaction or bases of low quality are trimmed from the raw read sequences to extract a sequence derived from the fragments of the sample DNA, thus generating read sequences. Or, raw read sequences may be directly used as read sequences if the trimming or the like is not needed. The amplified fragments from which the raw read sequences or read sequences derived may be a plurality of amplified fragments of at least some of the amplified fragments contained in the PCR products. The read sequences may be acquired for all of the amplified fragments contained in the PCR products, however, which is not required. One or more read sequences are generated for each of the plurality of amplified fragments. The one or more read sequences thus generated for each one of the amplified fragments contain sequence information on the amplified fragment (i.e., one of two complementary strands of one fragment of the sample DNA from which it derived). Thus, a plurality of read sequences is acquired as a result of sequencing the PCR products. Herein, data including the plurality of read sequences acquired at this stage are occasionally referred to as "sequencing data".

### (2-3) Grouping of read sequences

Subsequently, read sequences containing sequence information on the same region in the sample DNA are collected from the plurality of read sequences acquired, on the basis of sequence information on each read sequence. The collected read sequences are put into a group. Accordingly, a "group of read sequences" to be generated in the method according to the present invention is a group of read sequences containing sequence information on the same region in the sample DNA, in other words, a group of read sequences estimated to be derived from the same fragment of the sample DNA. In the method according to the present invention as defined in the claims, one or more groups of read sequences can be generated, in a manner depending on the number of fragments of sample DNA subjected to PCR and the amount of sequencing data.

In an aspect of the method according to the present invention as defined in the claims, one or more read sequences are generated for one amplified fragment contained in the PCR products, and the read sequences acquired are put into a group as described above. In a preferred aspect, read sequences to be used to generate each of the above-described groups of read sequences are read sequences containing information on the whole sequence of the original fragment of the sample DNA (i.e., the fragment of the sample DNA as an origin of amplified fragments from which the read sequences are derived). Examples of procedures to select the read sequences containing information on the whole sequence of an original fragment of the sample DNA out of the read sequences acquired through sequencing include a method of selecting read sequences with high reading accuracy (quality value) for a base at the end, and a method of sequencing amplified fragments each including a label sequence added at a terminal followed by selecting read sequences on the basis of the presence or absence of information on the label sequence. Among them, an example of the method using a label sequence will be described hereinafter in more detail: first, different label sequences are added to both terminals of each of the fragments of the sample DNA, and the resulting fragments of the sample DNA are subjected to PCR amplification to prepare amplified fragments each including the label sequences at both terminals; and the amplified fragments obtained are subjected to sequencing to acquire read sequences derived from each of the amplified fragments and information on the label sequences accompanying thereto. A read sequence accompanied by information on both of the label sequences at both terminals is regarded as a read sequence containing information on the whole sequence of the original fragment of the sample DNA. In another example, a label sequence is added to one terminal of each of the fragments of the sample DNA, and the resulting fragments of the sample DNA are subjected to PCR amplification to prepare amplified fragments each including the label sequence; and the amplified fragments obtained are subjected to sequencing from the respective terminals without the label sequence. A read sequence accompanied by information on the label sequence is regarded as a read sequence containing information on the whole sequence of the original fragment of the sample DNA. Here, the information on the label sequence(s) may be acquired from raw read sequences, or from sequence information on a sequencing primer.

Examples of ways to generate groups of read sequences from the collected read sequences include a method of collecting read sequences to be mapped on the same position in a reference sequence, and a method of collecting read sequences identical in sequences at least in both terminal regions. The phrase "identical in sequences at least in both terminal regions" means that the read sequences have sequence identity of 80% or higher, preferably 90% or higher, more preferably 95% or higher, even more preferably 97% or higher, at least in both terminal regions, and both terminals thereof are aligned at identical positions among the read sequences. The length of the "terminal region" can be appropriately selected, and, for example, may be a length of 10 bases or more, preferably of about 10 to 30 bases, with the terminal included. Alternatively, groups of read sequences may be generated by collecting read sequences such that the whole-sequence identity thereof is 80% or higher, preferably 90% or higher, more preferably 95% or higher, even more preferably 97% or higher and both terminals thereof are aligned at identical positions among the read sequences.

### (2-4) Extraction of sequence information on sample DNA from groups of read sequences

Next, sequence information on the sample DNA is extracted from the groups of read sequences acquired. Specifically, one sequence data is derived for each of the groups of read sequences by using information on read sequences included in the group. The sequence data acquired represents a consensus sequence for a certain fragment of the sample DNA from which the read sequences in the group are derived.

For example, one sequence data can be generated by building consensus of sequence information among read sequences included in a group of read sequences. Example of specific techniques to build consensus among read sequences include: a method in which read sequences are aligned, and if all the aligned read sequences have a common base at the same position, the base is regarded as a "consensus base"; a method in which read sequences are aligned, and the most frequent base is then determined at each position of the sequence and extracted as a "consensus base"; a method in which read sequences are aligned, and a base for which a sequencer provides the highest reading accuracy (quality value) among bases at the same position is employed as a "consensus base"; a method in which read sequences are aligned, and "consensus bases" are stochastically determined on the basis of quality values, frequencies of appearance of bases, and so forth; and a method of combination of any of them.

In building consensus among read sequences, all of the read sequences included in a group of read sequences may be used; however, only some of the read sequences in the group may be used. Building of consensus among read sequences can exclude errors including read errors in sequencing, thereby providing highly accurate reading results. The resulting sequence data can be acquired as final sequence data representing the sequence of a region of the sample DNA.

### (2-5) Sequencing based on information on complementary strands

Base variation due to oxidative modification of DNA or the like, which causes sequencing error, generally occurs only in one of two strands of DNA. Therefore, use of sequencing information on two complementary strands of DNA enables selective identification of true mutations fixed in the two strands, without detecting a base variation which occurred only in one strand as a mutation. The sequences of two complementary strands of DNA share equivalent information, in spite of their complementarity. Hence, information on complementary strands can be acquired, theoretically, by searching for sequences containing equivalent information from read sequences acquired through sequencing. If the sample DNA is prepared from a genome sequence of a certain biological species, for example, two read sequences which are derived from two complementary strands constituting a fragment of the sample DNA, and correspond to the same read region, are mapped on the same position in the genome when being mapped on a reference sequence for the biological species which is a subject of analysis. Therefore, read sequences derived from each of two complementary strands can be acquired by collecting read sequences to be mapped on the same position of the genome, and screening the collected read sequences with consideration of complementary strands from which they are derived. Further, consensus is built among the read sequences derived from each of two complementary strands, and thus highly accurate read information reflecting information on a complementary strand can be acquired.

Accordingly, the present invention provides as defined in the claims, a method for sequencing DNA using information on complementary strands. In the method, read sequences are generated for each of two complementary strands constituting each of the fragments of the sample DNA in sequencing the PCR products described in (2-2) above. More specifically, in generating read sequences for each of the plurality of amplified fragments contained in the PCR products by sequencing the PCR products, one or more read sequences are generated for each of amplified fragments derived from each of two complementary strands constituting each of the fragments of the sample DNA. That is, two or more read sequences are acquired for one fragment of the sample DNA, and the read sequences contain sequence information on one and the other of two complementary strands of the fragment of the sample DNA from which the read sequences derived.

Subsequently, one or more groups of read sequences are generated from the plurality of read sequences acquired. The way to generate groups of read sequences is as described in (2-3) above. Groups of read sequences to be acquired here each include read sequences containing sequence information on one and the other of two complementary strands of a certain fragment of the sample DNA. Therefore, sequence data reflecting information on complementary strands can be generated by building consensus of sequence information among read sequences included in each of the groups of read sequences. The technique to build consensus among read sequences is specifically as described in (2-4) above. In building consensus among read sequences, all of the read sequences included in a group of read sequences may be used; however, only some of the read sequences included in the group may be used.

The step of building consensus among read sequences preferably includes collecting, from a group of read sequences, at least one read sequence derived from each of two complementary strands of a fragment of the sample DNA, and building consensus of sequence information among the collected read sequences. Thereby, consensus data using information on complementary strands (herein, also referred to as "consensus read sequence considering complementary strands") can be acquired. The consensus read sequence considering complementary strands thus acquired, which is a highly accurate reading in which errors which occur only in one strand such as read errors in sequencing or errors due to oxidative modification of DNA or the like are excluded, can be acquired as final sequence data representing the sequence of one fragment of the sample DNA.

Examples of procedures to collect read sequences derived from each of two complementary strands of a fragment of the sample DNA include the following: a label sequence, which allows two complementary strands of each of the fragments of the sample DNA to be distinguished, is added to the fragments of the sample DNA in advance to prepare amplified fragments each including the label sequence; the amplified fragments are then subjected to sequencing to acquire read sequences derived from each of the amplified fragments and information on the label sequence accompanying thereto; groups of read sequences are generated from the read sequences acquired; and read sequences derived from each of strands complementary to each other are then collected from each of the groups of read sequences with use of the information on the label sequence accompanying to each read sequence.

When a label sequence is added to each of the fragments of the sample DNA in the aforementioned procedure, the label sequence labels each of read sequences derived from two complementary strands of each of the fragments of the sample DNA in such a manner that read sequences derived from one and the other complementary strands are distinguished each other. However, the label sequence does not need to identify which fragment of the sample DNA a read sequence is derived from. Preferably, label sequences added to each of the fragments of the sample DNA are identical. For example, two complementary strands constituting each of the fragments of the sample DNA are each allowed to include different label sequences in the 5'-terminal side and the 3'-terminal side. Sequencing the amplified fragments imparts read sequences derived from the amplified fragments and information on label sequences included in the amplified fragments each accompanying to each of the read sequences. It is preferable that the label sequences in the 5'-terminal side are identical and the label sequences in the 3'-terminal side are also identical between one and the other strand of the fragments of the sample DNA, and both strands of label sequence regions added to both terminals of each of the fragments of the sample DNA include sequences not complementary to each other. Preferably, a common label sequence is used for the 5'-terminal side of each of the fragments of the sample DNA, and a common label sequence is used for the 3'-terminal side of each of the fragments of the sample DNA. As a result, two single strands constituting each of the fragments of the sample DNA can each include different label sequences in the 5'-terminal side and the 3'-terminal side, and the label sequences included in the 5'-terminal side are in common and the label sequences included in the 3'-terminal side are in common among each of single strands. Examples of label sequences which allow identifying which of two complementary strands of a fragment of the sample DNA a read sequence is derived from include adapter sequences attached to a TruSeq from Illumina, Inc. By sequencing amplified fragments obtained from fragments of the sample DNA each including the label sequences added thereto, read sequences derived from the amplified fragments and information on label sequences accompanying thereto can be acquired.

Next, a preferred procedure in collecting read sequences derived from each of strands complementary to each other from a group of read sequences by using information on the label sequences will be described. The read sequences included in a group of read sequences are mapped on a reference sequence. A read sequence accompanied by information on the label sequence in the 5'-terminal side and positioned in the reference sequence such that the beginning is positioned in the 5'-terminal side to the end, and a read sequence accompanied by information on the label sequence in the 3'-terminal side and positioned in the reference sequence such that the beginning is positioned in the 3'-terminal side to the end are derived from the same one single strand of two complementary strands of a fragment of the sample DNA. On the other hand, a read sequence accompanied by information on the label sequence in the 3'-terminal side and positioned in the reference sequence such that the beginning is positioned in the 5'-terminal side to the end, and a read sequence accompanied by information on the label sequence in the 5'-terminal side and positioned in the reference sequence such that the beginning is positioned in the 3'-terminal side to the end are derived from the other single strand of two complementary strands of the fragment of the sample DNA. Thus, which of two complementary strands constituting a fragment of the sample DNA each read sequence in a group of read sequences is derived from can be identified on the basis of the location in a reference sequence of the read sequence mapped on the reference sequence and information on the accompanying label sequences. Alternatively, sequencing reaction which is initiated only when a specific label sequence has been added to a terminal of an amplified fragment allows identification of a read sequence derived from a certain single strand of each fragment of the sample DNA on the basis of information on the label sequence. Thus, read sequences derived from each of strands complementary to each other can be collected from a group of read sequences by identifying in advance read sequences derived from the same one single strand of a fragment of the sample DNA.

An example of detail procedures to acquire a consensus read sequence considering complementary strands from each of the groups of read sequences as described above includes a procedure in which two read sequences each derived from each of two complementary strands of a fragment of the sample DNA are selected from a group of read sequences and consensus of sequence information is built between the two read sequences. This procedure may be repeated to generate a plurality of consensus read sequences considering complementary strands, among which consensus may be built to generate one consensus read sequence considering complementary strands. Another example of detail procedures to acquire such a consensus read sequence considering complementary strands includes a procedure in which read sequences included in a group of read sequences are classified into groups derived from one and the other of two complementary strands of a fragment of the sample DNA, and consensus is built among read sequences in each group, and consensus is further built between the resulting two consensus data to generate one consensus read sequence considering complementary strands. Still another example includes a procedure in which consensus is built among read sequences included in a group of read sequences to generate a consensus read sequence, without distinguishing read sequences derived from one of two complementary strands of a fragment of the sample DNA from those derived from the other.

### (2-6) Extraction of sequence information on sample DNA using read pairs

In an embodiment of the method according to the present invention as defined in the claims, one pair of read sequences consisting of two read sequences (i.e., "read pair") is generated, instead of one read sequence, for each of the plurality of amplified fragments contained in the PCR products in sequencing of the PCR products described in (2-2) above. From the read pairs generated, sequence information on the sample DNA is extracted on the basis of the above principle.

In the present method, one or more read pairs are generated for each of the amplified fragments through sequencing of the PCR products. The generation of one or more read pairs is performed for each of two or more amplified fragments derived from each of the fragments of the sample DNA. The read pairs contain sequence information on either one of two complementary strands of the corresponding fragment of the sample DNA. Thus, in an aspect, the plurality of read sequences acquired in the above-described sequencing of the PCR products includes a plurality of read pairs.

One of two read sequences constituting each of the read pairs is a read sequence containing sequence information on reading out of the sequence of one strand of two complementary strands constituting the amplified fragment, from the 5'-terminal side to the 3'-terminal side (referred to as "read 1"), and the other is a read sequence containing sequence information on reading out of the sequence of the same one strand from the 3'-terminal side to the 5'-terminal side (referred to as "read 2"). Read 1 and read 2 are disposed in the opposite direction with respect to the original strand (a single strand constituting the amplified fragment). Specifically, when being mapped on the original strand, the beginning of read 1 is positioned in the 5'-terminal side to the end in the original strand, and the beginning of read 2 is, on the other hand, positioned in the 3'-terminal side to the end in the original strand (see Schematic Diagram 1 shown later).

Subsequently, read pairs containing sequence information on the same region in the sample DNA are selected out of the plurality of read pairs in the acquired sequencing data. The read pairs collected are put into a group. Examples of ways to generate such groups of read pairs include a method of mapping read 1 and read 2 in each read pair on a reference sequence and collecting read sequence pairs into the same group such that they have the same region between the beginning of read 1 and the beginning of read 2 in the reference sequence. In an example of more detailed procedures, first, read pairs whose positions of the beginning of one read sequence (read 1 or 2) included therein are the same in a reference sequence are collected, and from the collected read sequence pairs, read sequence pairs whose positions of the beginning of the other read sequence (read 2 or 1) therein are the same in the reference sequence are collected into the same group.

Thus, a "group of read sequence pairs (read pairs)" to be generated in the method according to the present invention as defined in the claims is a collection of read pairs estimated to contain sequence information on the same region in the sample DNA (i.e., derived from the same fragment of the sample DNA). In the method according to the present invention as defined in the claims, one or more groups of read pairs can be generated, in a manner depending on the number of fragments of the sample DNA subjected to PCR and the amount of sequencing data.

Subsequently, sequence information on the sample DNA is extracted by using information on read sequences included in the groups of read pairs acquired. For example, one sequence data can be generated by building consensus of sequence information among read sequences included in a group of read pairs. The detail procedure to build consensus among read sequences is as described in (2-4) above. In building consensus among read sequences, the read sequences of all of the read pairs included in a group of read pairs may be used; however, the read sequences of only some of the read pairs included in the group may be used. The resulting sequence data can be acquired as final sequence data representing the sequence of a fragment of the sample DNA.

### (2-7) Sequencing with read pairs based on information on complementary strands

A method for sequencing DNA using information on complementary strands can be implemented with the above-described read pairs. In this method, one or more read pairs are generated for each of amplified fragments derived from each of two complementary strands constituting each of the fragments of the sample DNA in the sequencing of the PCR product as described in (2-6) above. That is, two or more read pairs are acquired for one fragment of the sample DNA, and the read pairs contain sequence information on one and the other of two complementary strands of the corresponding fragment of the sample DNA. Hence, the plurality of read sequences acquired through the above-described sequencing includes a plurality of read pairs in the present aspect.

Subsequently, one or more groups of read pairs are generated from the plurality of read pairs acquired. The way to generate groups of read pairs is as described in (2-5) above. Groups of read pairs to be acquired here each include read pairs containing sequence information on one and the other of two complementary strands of a certain fragment of the sample DNA. Therefore, sequence data reflecting information on complementary strands can be generated by building consensus of sequence information among read sequences included in each of the groups of read pairs. The detail procedure to build consensus among read sequences is as described in (2-4) above. In building consensus among read sequences, the read sequences of all of the read pairs included in a group of read pairs may be used; however, the read sequences of only some of the read pairs included in the group may be used.

Next, one sequence data is derived by using information on read sequences included in each of the groups of read pairs acquired. For example, one piece of sequence data can be generated by building consensus of sequence information among read sequences included in a group of read pairs. The sequence data acquired represents the sequence of a certain fragment of the sample DNA from which the read sequences in the group are derived. If read sequences containing sequence information on two complementary strands of a fragment of the sample DNA are included in a group of read pairs, building of consensus thereamong can exclude errors which occur only in one strand such as read errors in sequencing or errors due to oxidative modification of DNA or the like.

The step of building consensus among read sequences included in a group of read pairs preferably includes collecting at least one read pair derived from each of two complementary strands of a fragment of the sample DNA from a group of read pairs, and building consensus of sequence information among read sequences included in the collected read pairs. Thereby, a consensus read sequence considering complementary strands can be acquired. The resulting consensus read sequence considering complementary strands can be acquired as final sequence data representing the sequence of a fragment of the sample DNA.

Examples of procedures to collect read pairs derived from each of two complementary strands of a fragment of the sample DNA from a group of read pairs include the following: a label sequence, which allows two complementary strands of each of the fragments of the sample DNA to be distinguished, is added to the fragments of the sample DNA in advance to prepare amplified fragments each including the label sequence; the amplified fragments are then subjected to sequencing to acquire read pairs derived from each of the amplified fragments and information on the label sequence accompanying thereto; groups of read pairs are generated from the read pairs acquired; and read pairs derived from each of strands complementary to each other are then collected from each of the groups of read pairs with use of the information on the label sequence accompanying to each read pair.

When a label sequence is added to each of the fragments of the sample DNA in the aforementioned procedure, for example, different label sequences are included in the 5'-terminal side and the 3'-terminal side of the two complementary strands constituting each of the fragments of the sample DNA. It is preferable that the label sequences in the 5'-terminal side are identical and the label sequences in the 3'-terminal side are also identical between one and the other strand of the fragments of the sample DNA, and both strands of label sequence regions added to both terminals of each of the fragments of the sample DNA include sequences not complementary to each other. Preferably, a common label sequence is used for the 5'-terminal side of each of the fragments of the sample DNA, and a common label sequence is used for the 3'-terminal side of each of the fragments of the sample DNA. As a result, two single strands constituting each of the fragments of the sample DNA can each include different label sequences in the 5'-terminal side and the 3'-terminal side, and the label sequences included in the 5'-terminal side are in common and the label sequences included in the 3'-terminal side are in common among each of single strands. Examples of label sequences which allow identifying which of two complementary strands of a fragment of the sample DNA a read sequence is derived from include adapter sequences attached to a TruSeq from Illumina, Inc. By sequencing amplified fragments obtained from fragments of the sample DNA each including the label sequences added thereto, read pairs derived from the amplified fragments and information on the label sequences accompanying to each read sequence included therein can be acquired. In this case, either one of read 1 or read 2 in each read pair is accompanied by information on the label sequence in the 5'-terminal side, and the other is accompanied by information on the label sequence in the 3'-terminal side.

Next, a preferred procedure in collecting read pairs derived from each of strands complementary to each other from a group of read pairs by using information on the label sequences will be described with reference to Scheme 1 below. The read pairs included in a group of read pairs are mapped on a reference sequence. A read pair (in Scheme 1, read pair consisting of A-R1* and A-R2^{X}) such that the beginning of the read sequence accompanied by information on the label sequence in the 5'-terminal side is positioned in the 5'-terminal side to the beginning of the other read sequence in the reference sequence (i.e., the beginning of the read sequence accompanied by information on the label sequence in the 3'-terminal side is positioned in the 3'-terminal side to the beginning of the other read sequence in the reference sequence), and a read pair (in Scheme 1, read pair consisting of B-R1* and B-R2^{X}) such that the beginning of the read sequence accompanied by information on the label sequence in the 5'-terminal side is positioned in the 3'-terminal side to the beginning of the other read sequence in the reference sequence (i.e., the beginning of the read sequence accompanied by information on the label sequence in the 3'-terminal side is positioned in the 5'-terminal side to the beginning of the other read sequence in the reference sequence) are distinguished from each other. As shown in Scheme 1 below, the former read pair and the latter read pair are derived from two complementary strands (strand A and strand B) of a fragment of the sample DNA. Thus, which of two complementary strands constituting a fragment of the sample DNA each read pair in a group of read pairs is derived from can be identified on the basis of information on the label sequences accompanying to two read sequences included in the read pair and positional relation between the two read sequences in a reference sequence. Alternatively, sequencing reaction which is initiated only when a specific label sequence has been added to a terminal of an amplified fragment allows identification of a read pair derived from a certain single strand of each fragment of the sample DNA on the basis of information on the label sequence. Thus, read pairs derived from each of strands complementary to each other can be collected from a group of read pairs by identifying in advance read pairs derived from the same one single strand of a fragment of the sample DNA.

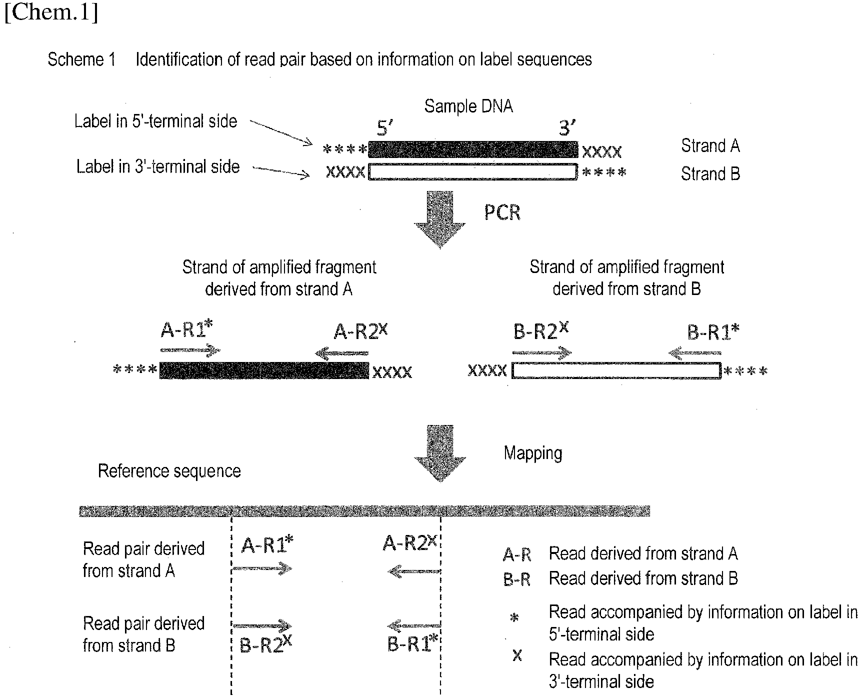

An example of detail procedures to acquire a consensus read sequence considering complementary strands from each of the groups of read pairs as described above includes a procedure in which two read pairs each derived from each of two complementary strands of a fragment of the sample DNA are selected from a group of read pairs and consensus of sequence information is built among read sequences included in the read pairs. This procedure may be repeated to generate a plurality of consensus read sequences considering complementary strands, among which consensus may be built to generate one consensus read sequence considering complementary strands. Another example of detail procedures to acquire such a consensus read sequence considering complementary strands includes a procedure in which read pairs included in a group of read pairs are classified into groups derived from one and the other of two complementary strands of a fragment of the sample DNA, and consensus is built among read sequences included in read pairs of each group and consensus is further built between the resulting two consensus data to generate one consensus read sequence considering complementary strands. Still another example includes a procedure in which consensus is built among read sequences included in a group of read pairs to generate a consensus read sequence, without distinguishing read sequences derived from one of two complementary strands of a fragment of the sample DNA from those derived from the other. An example of more detail procedures to generate a consensus read sequence considering complementary strands from a group of read pairs is described later in Example 1 (Schematic Diagram 3).

In the method according to the present invention as defined in the claims, the above-described grouping of read sequences or read pairs can be performed on the basis of sequence information on the sample DNA itself contained in read sequences. In this method, read sequences containing sequence information on strands complementary to each other can be distinguished, for example, on the basis of sequence information on label sequences added to each fragment of the sample DNA.
On the other hand, it is not needed to identify which fragment of the sample DNA a read sequence is derived from by labeling. Accordingly, each of the label sequences to be used in this method is not required to be a label which allows individual identification of fragments of the sample DNA. In this method it is not needed to add a label for individually identifying fragments of the sample DNA (e.g., a tag sequence specific to each individual fragment of the sample DNA as described in Non Patent Literature 8 and Patent Literature 1). In the method according to the present invention as defined in the claims, identical label sequences may be used for all the fragments as long as the label sequences allow identifying which of two complementary strands each of read sequences in a group of read sequences or read pairs are derived from. Hence, the method according to the present invention allows highly accurate sequencing with library preparation and experimental operations almost the same as those in sequencing methods that have generally been performed in the art.

### (3. Optimization of sequencing conditions)

In the above-described method for sequencing DNA according to the present invention as defined in the claims, due to the lack of use of labels for individually identifying fragments of the sample DNA, there is the possibility of misrecognizing sequences actually derived from different DNA fragments as those derived from the same DNA fragment, which may lead to the possibility that mutations to be detected in proper cases are regarded as errors and missed. When diagnosis is performed on the basis of the presence or absence of a mutation in a specific region such as cancer genes, for example, accurate identification of mutations is required, and missing of a mutation can be a serious problem. In the case of mutagenicity evaluation for chemicals or estimation of mutagen on the basis of information on mutations generated in the whole genome of a certain individual, however, understanding of overview of mutations in the whole sample DNA is more important, and accurate identification of mutations in a specific region is not necessarily required. In this case, missing of a mutation may be acceptable, unless such missing occurs so frequently that the tendency of mutation in the whole sample DNA is affected.

Regarding optimization of sequencing conditions, Kennedy et al. have previously examined optimization of PCR conditions in library preparation and the amount of sequencing data in a Duplex Sequencing method (Nature protocol, 2014 9(11):2586-2606). For example, the examination made by Kennedy et al. has proposed the requirement that at least three or more read sequences derived from the same DNA fragment are included in generating a consensus read sequence for each PCR product. However, this optimum conditions are designed for the purpose of accurate understanding of the presence or absence of any mutation in a targeted genome region, and are probably different from optimum conditions in the method according to the present invention purposing general understanding of the tendency of mutations in the whole sample DNA. In understanding overview of mutations in the whole sample DNA in the method according to the present disclosure, the large numbers of read sequences derived from the same DNA fragment is not required in order to achieve a required accuracy. Rather, it is more efficient to evaluate an increased number of different DNA fragments while the number of reads derived from the same DNA fragment is reduced. Hence, this method probably allows analysis of the tendency of mutations in the whole sample DNA with a less sequence amount than under the conditions proposed by Kennedy et al.

As described later in Example 2, optimum conditions and applicable conditions to achieve acceptable accuracy for analysis of the tendency of mutations in sample DNA were studied for sequencing DNA. Described in the following are under what conditions and with what frequency missing of a mutation occurs in the method for sequencing DNA according to the present invention as defined in the claims, and sequencing conditions to avoid such missing.

Factors which can affect the efficiency and accuracy of a sequencing method are as follows.

### [1] Efficiency of acquiring sequence data for sample DNA (data efficiency)

In the sequencing method according to the present invention, PCR is performed for fragments of sample DNA, and the products are used as a library (a mixture of PCR products from various DNA fragments) and subjected to sequencing. Hence, the efficiency of acquiring sequence data for the sample DNA (e.g., consensus data for each group of read sequences or a consensus read sequence considering complementary strands) from sequencing data (data efficiency) depends on fractions of PCR products (including forward strands and complementary strands) derived from the same DNA fragment in the library and the amount of sequencing data (the number of reads or bp) from the library. Thus, appropriate PCR conditions and setting of the amount of sequencing data are important for data efficiency.

In addition, the amount of input DNA in PCR and the amount of sequencing data affect the number of read sequences or read pairs included in each of the above-described groups of read sequences or read pairs. If the number of read sequences or read pairs included in each group of read sequences or read pairs is decreased, the probability of successful generation of sequence data for sample DNA decreases to lower the data efficiency. If the number of read sequences or read pairs per group is excessively large, however, the number of reads to be used to generate one sequence data is excessively large, lowering the data efficiency, similarly.

### [2] Frequency of misrecognizing different DNA fragments as identical fragments (misrecognition of fragments)

Misrecognition of fragments can occur when two fragments derived from different fragments of sample DNA and involving overlapping in their sequences are included in a library and the fragments both have been subjected to sequencing. Hence, misrecognition of fragments is associated with the diversity of DNA sequences in a library (the amount of input DNA in PCR). The diversity of DNA sequences in a library is affected by the diversity of the sequence of sample DNA, and the diversity of the sequence of sample DNA generally depends on the size of the sample DNA. Therefore, the size of sample DNA also has influence on misrecognition of fragments.

Misrecognition of fragments depends on the fraction of read sequences or read pairs not derived from the same fragment of sample DNA which are included in each of the above-described groups of read sequences or read pairs, and the fraction is determined from the ratio between (1) the probability that PCR products derived from the same fragment of sample DNA in a library are subjected to sequencing and (2) the probability that PCR products derived from different DNA fragments the sequences of which happen to be identical (or have such high identity that they are regarded to be identical) are subjected to sequencing. If the amount of input DNA in PCR is decreased and the number of PCR cycles is increased, fractions of products each derived from the same fragment in the total of the PCR products increase, which increases the probability of (1) and inversely decreases the probability of (2), and hence misrecognition of fragments is less likely to occur. Meanwhile, the ratio between (1) and (2) is independent of the amount of sequencing data, and thus the frequency of misrecognition of fragments does not depend on the amount of sequencing data.

From [1] and [2] above, PCR conditions in library preparation, in particular, the amount of input DNA in PCR, the amount of sequencing data, and the size of sample DNA are primarily important as factors which can affect the efficiency and accuracy of sequencing. Moreover, the number of read sequences or read pairs included in each group of read sequences or read pairs, which depends on those factors, can serve as an index to determine the efficiency and accuracy of sequencing.

Appropriate amount for input DNA in PCR can depend on the size of sample DNA. If the amount of input DNA in PCR is excessively large for sample DNA of small size, the probability of misrecognition of fragments is higher, possibly leading to failure in correctly detecting mutations. If the amount of input DNA in PCR is large for sample DNA of large size, a huge amount may be needed for sequence data, in view of data efficiency described later. If the amount of input DNA in PCR is excessively small for sample DNA of large size, on the other hand, the diversity of the sequence of the sample DNA is not sufficiently reflected, possibly leading to a lower genome coverage. If the comprehensiveness of sequence data for the whole sequence of sample DNA does not matter, however, use of a small amount of input DNA in PCR as compared with the size of sample DNA is acceptable, unless the data efficiency is lowered. Possible examples include the case that partial regions are randomly selected from the whole sample DNA and mutation frequencies for the partial regions are studied. Accordingly, from the viewpoints of the probability of misrecognition of fragments and genome coverage, the amount of input DNA in PCR with respect to the size of sample DNA in the sequencing method according to the present invention as defined in the claims can be selected from an appropriate range according to the purpose of study. In light of reduction of the probability of misrecognition of fragments and an appropriate amount of sequence data, the amount of input DNA in PCR in the sequencing method according to the present invention is preferably 1.7 amol or less per 1 Mbp of sample DNA. For ensuring the comprehensiveness for genomes, on the other hand, the amount of input DNA in PCR is preferably 0.002 amol or more, even more preferably 0.005 amol or more, even more preferably 0.01 amol or more, even more preferably 0.03 amol or more, even more preferably 0.05 amol or more, even more preferably 0.1 amol or more, even more preferably 0.3 amol or more, even more preferably 1 amol or more per 1 Mbp of sample DNA.

In an example described, the amount of input DNA in PCR in the sequencing method is as follows: for bacteria, the genome size of which is approximately 5 Mbp, the amount of input DNA in PCR is preferably 0.1 to 250 amol, more preferably 0.3 to 250 amol, even more preferably 1 to 250 amol, even more preferably 2 to 125 amol, even more preferably 3.9 to 62.5 amol, and even more preferably 7.8 to 31.3 amol, per 1 Mbp of sample DNA; for yeasts, the genome size of which is approximately 10 Mbp, the amount of input DNA in PCR is preferably 0.05 to 250 amol, more preferably 0.1 to 250 amol, even more preferably 0.3 to 125 amol, even more preferably 1 to 62.5 amol, and even more preferably 2 to 31.3 amol, per 1 Mbp of sample DNA; for nematodes, the genome size of which is approximately 100 Mbp, the amount of input DNA in PCR is preferably 0.005 to 31.3 amol, more preferably 0.01 to 31.3 amol, even more preferably 0.03 to 15.6 amol, even more preferably 0.1 to 7.8 amol, and even more preferably 0.3 to 3.9 amol, per 1 Mbp of sample DNA; and for mice, the genome size of which is approximately 3 Gbp, the amount of input DNA in PCR is preferably 0.0003 to 1.7 amol, more preferably 0.0007 to 1.7 amol, even more preferably 0.002 to 1.7 amol, even more preferably 0.005 to 0.83 amol, even more preferably 0.01 to 0.42 amol, and even more preferably 0.03 to 0.21 amol, per 1 Mbp of sample DNA. Herein, the amount of input DNA in PCR reaction is the amount of DNA in a DNA sample to be used for PCR reaction, and does not include the amount of DNA from PCR primers or the like.

Further, appropriate amount for input DNA in PCR can depend on the amount of data (sequence information) available for analysis. If the amount of input DNA in PCR is excessively small, the number of fragments of sample DNA contained therein is smaller, leading to failure in acquiring sufficient data (sequence information) available for analysis. The amount of input DNA in PCR in the sequencing method described is preferably 0.1 amol or more, more preferably 1 amol or more, even more preferably 5 amol or more, even more preferably 20 amol or more, even more preferably 39 amol or more, and even more preferably 78 amol or more. For data efficiency, on the other hand, the amount of input DNA in PCR is preferably 100,000 amol or less, more preferably 20,000 amol or less, and even more preferably 5,000 amol or less. For example, the amount of input DNA in PCR in the sequencing method described is preferably 0.1 to 100,000 amol, more preferably 1 to 100,000 amol, even more preferably 5 to 100,000 amol, even more preferably 20 to 100,000 amol, even more preferably 20 to 20,000 amol, even more preferably 39 to 20,000 amol, even more preferably 78 to 20,000 amol, even more preferably 20 to 5,000 amol, even more preferably 39 to 5,000 amol, and even more preferably 78 to 5,000 amol.

In the sequencing method according to the present invention as defined in the claims, appropriate amount for sequencing data can depend on the amount of input DNA in PCR. A amount of sequencing data which is excessively large or excessively small with respect to the amount of input DNA in PCR results in lowered data efficiency. The amount of sequencing data in the sequencing method according to the present invention as the number of read pairs or the number of read sequences per 1 amol of the amount of input DNA in PCR is preferably 0.02 * 10⁶ (4 Mbp, as the amount of base pairs in read sequences or read pairs wherein the mean of the lengths of read sequences or of the total lengths of read sequences included in read pairs is 200 bp, and the value can vary depending on the mean of the lengths of read sequences or of the total lengths of read sequences included in read pairs, the same applies hereinafter) or larger, more preferably 0.04 * 10⁶ (8 Mbp) or larger, even more preferably 0.08 * 10⁶ (16 Mbp) or larger, even more preferably 0.16 * 10⁶ (32 Mbp) or larger, and, preferably 10 * 10⁶ (2,000 Mbp) or smaller, more preferably 5 * 10⁶ (1,000 Mbp) or smaller, even more preferably 2.5 * 10⁶ (500 Mbp) or smaller, even more preferably 2 * 10⁶ (400 Mbp) or smaller. For example, the amount of sequencing data in the sequencing method according to the present invention as defined in the claims as the number of read pairs or the number of read sequences per 1 amol of the amount of input DNA in PCR is preferably 0.02 to 10 * 10⁶ (4 to 2,000 Mbp), more preferably 0.04 to 5 * 10⁶ (8 to 1,000 Mbp), even more preferably 0.08 to 2.5 * 10⁶ (16 to 500 Mbp), and even more preferably 0.16 to 2 * 10⁶ (32 to 400 Mbp).

The amount of input DNA in PCR and the amount of sequencing data each affect the number of read sequences or read pairs included in each group of read sequences or read pairs. Meanwhile, the mean of the numbers of read sequences or read pairs included in groups of read sequences or read pairs under conditions providing the maximum data efficiency is almost constant among different conditions for the amount of input DNA in PCR and the amount of sequencing data (see Table 3 shown later in Examples). Therefore, sequencing with an optimum data efficiency and accuracy may be achieved in the sequencing method according to the present invention as defined in the claims through establishing conditions so that the mean of the numbers of read sequences or read pairs included in groups of read sequences or read pairs falls within a specific range. In the sequencing method according to the present invention as defined in the claims, the number of read sequences included in each group of read sequences, or the number of read pairs included in each group of read pairs is, as the mean among the groups, preferably 1.05 or larger, more preferably 1.1 or larger, even more preferably 1.2 or larger, even more preferably 1.4 or larger, and, preferably 30 or smaller, more preferably 20 or smaller, even more preferably 10 or smaller, even more preferably 5 or smaller. In the sequencing method according to the present invention as defined in the claims, the number of read sequences or read pairs included in each group of read sequences or read pairs is, as the mean among the groups, preferably 1.05 to 30, more preferably 1.1 to 20, even more preferably 1.2 to 10, and even more preferably 1.4 to 5.

From the above description, appropriate amount for sequencing data can also depend on the size of sample DNA. Larger amount of input DNA in PCR is needed for sample DNA of large size to achieve sufficiently high genome coverage therefor. Hence, if the amount of sequencing data is excessively small for the size of sample DNA, a sufficient number of read pairs to generate sequence data cannot be acquired, possibly leading to lowered data efficiency. If the comprehensiveness of sequence data for the whole sequence of sample DNA does not matter, however, use of a small amount of sequencing data as compared with the size of sample DNA is acceptable, unless the data efficiency is lowered. For sample DNA of small size, on the other hand, high genome coverage is achieved with a smaller amount of input DNA in PCR. Thus, the data efficiency is lowered if the amount of sequencing data is excessively large for the size of sample DNA. The amount of sequencing data as the number of read sequences or read pairs per 1 Mbp of sample DNA in the sequencing method according to the present invention as defined in the claims is preferably 0.05 * 10⁶ (10 Mbp) or larger, more preferably 0.1 * 10⁶ (20 Mbp) or larger, even more preferably 0.2 * 10⁶ (40 Mbp) or larger, even more preferably 0.5 * 10⁶ (100 Mbp) or larger, even more preferably 1 * 10⁶ (200 Mbp) or larger, even more preferably 2 * 10⁶ (0.4 Gbp) or larger, and, preferably 1,600 * 10⁶ (320 Gbp) or smaller, more preferably 800 * 10⁶ (160 Gbp) or smaller, even more preferably 400 * 10⁶ (80 Gbp) or smaller, even more preferably 200 * 10⁶ (40 Gbp) or smaller, even more preferably 100 * 10⁶ (20 Gbp) or smaller, even more preferably 50 * 10⁶ (10 Gbp) or smaller. For example, the amount of sequencing data as the number of read sequences or read pairs per 1 Mbp of sample DNA in the sequencing method according to the present invention is preferably 0.05 to 1,600 * 10⁶ (0.01 to 320 Gbp), more preferably 0.1 to 800 * 10⁶ (0.02 to 160 Gbp), even more preferably 0.2 to 400 * 10⁶ (0.04 to 80 Gbp), even more preferably 0.5 to 200 * 10⁶ (0.1 to 40 Gbp), even more preferably 1 to 100 * 10⁶ (0.2 to 20 Gbp), and even more preferably 2 to 50 * 10⁶ (0.4 to 10 Gbp). If the size of sample DNA is large, for example, as with the case of genomic DNA derived from a mammal and the comprehensiveness of sequence data for the whole sequence of sample DNA does not matter, the amount of sequencing data in the sequencing method according to the present invention as defined in the claims may be smaller than 0.05 * 10⁶ (10 Mbp), as the number of read sequences or read pairs per 1 Mbp of sample DNA. For mice, the genome size of which is approximately 3 Gbp, for example, the amount of sequencing data as the number of read sequences or read pairs per 1 Mbp of sample DNA is preferably 0.00003 to 16 * 10⁶ (0.006 to 3,200 Mbp), more preferably 0.00007 to 8 * 10⁶ (0.014 to 1,600 Mbp), even more preferably 0.0001 to 4 * 10⁶ (0.02 to 800 Mbp), even more preferably 0.0003 to 2 * 10⁶ (0.06 to 400 Mbp), even more preferably 0.0005 to 1 * 10⁶ (0.1 to 200 Mbp), and even more preferably 0.001 to 0.5 * 10⁶ (0.2 to 100 Mbp).

The efficacy of the sequencing method according to the present invention as defined in the claims can depend on the size of sample DNA. If the size of sample DNA is excessively small, the diversity of sequences in a library for sequencing is lower and the probability of misrecognition of fragments increases. This indicates that reduction of the size of sample DNA requires smaller amount for input DNA in PCR, resulting in a smaller amount of final sequence data to be acquired. Hence, the sequencing method when applied to genomic mutation analysis, may fail in analyzing a sufficient amount of mutation data depending on the size of sample DNA. The size of sample DNA in the sequencing method according to the present invention as defined in the claims is preferably 10 kbp or more, more preferably 100 kbp or more, even more preferably 1 Mbp or more, and even more preferably 4 Mbp or more. The size of sample DNA is preferably the size of genomic DNA of an organism from which the sample DNA is derived, and more preferably the size of DNA in a region applicable to sequencing in the genomic DNA. On the other hand, it is also preferable that the size of sample DNA is the size of DNA in a region of a target of sequencing in the genomic DNA or the size of DNA in a region of target of analysis in the genomic DNA. Examples of the region applicable to sequencing include regions applicable to PCR reaction or sequencing reaction, examples of the region of a target of sequencing include exon regions in cells of mammals, or the like, and examples of the region of target of analysis include regions in a reference sequence to be used for analysis and regions of reference sequences for which groups of read sequences or read pairs have been successfully generated.

In an aspect of the sequencing method described herein, the size of sample DNA is approximately 5 Mbp, the amount of input DNA in PCR is preferably 10 to 1,250 amol, and the amount of sequencing data as the number of read sequences or read pairs is 0.2 to 12,500 * 10⁶ (0.04 to 2,500 Gbp), preferably 0.4 to 6,250 * 10⁶ (0.08 to 1,250 Gbp), more preferably 0.8 to 3,125 * 10⁶ (0.16 to 625 Gbp), and even more preferably 1.6 to 2,500 * 10⁶ (0.32 to 500 Gbp). More preferably, the size of sample DNA is approximately 5 Mbp, the amount of input DNA in PCR is 20 to 625 amol, and the amount of sequencing data as the number of read sequences or read pairs is 0.4 to 6,250 * 10⁶ (0.08 to 1,250 Gbp), preferably 0.8 to 3,125 * 10⁶ (0.16 to 625 Gbp), more preferably 1.6 to 1,563 * 10⁶ (0.32 to 313 Gbp), and even more preferably 3.2 to 1,250 * 10⁶ (0.64 to 250 Gbp). Even more preferably, the size of sample DNA is approximately 5 Mbp, the amount of input DNA in PCR is 39 to 313 amol, and the amount of sequencing data as the number of read sequences or read pairs is 0.78 to 3,130 * 10⁶ (0.156 to 626 Gbp), preferably 1.56 to 1,565 * 10⁶ (0.312 to 313 Gbp), more preferably 3.12 to 783 * 10⁶ (0.624 to 157 Gbp), and even more preferably 6.24 to 626 * 10⁶ (1.248 to 125 Gbp). In another aspect of the sequencing method described herein invention, the size of sample DNA is approximately 5 Mbp, and the number of read sequences or read pairs per group of read sequences or read pairs is, as the mean among the groups, 1.05 to 30, preferably 1.1 to 20, even more preferably 1.2 to 10, and even more preferably 1.4 to 5.

In still another aspect, the size of sample DNA is approximately 3 Gbp, the amount of input DNA in PCR is preferably 10 to 5,000 amol, and the amount of sequencing data as the number of read sequences or read pairs is 0.2 to 50,000 * 10⁶ (0.04 to 10,000 Gbp), preferably 0.4 to 25,000 * 10⁶ (0.08 to 5,000 Gbp), more preferably 0.8 to 12,500 * 10⁶ (0.16 to 2,500 Gbp), and even more preferably 1.6 to 10,000 * 10⁶ (0.32 to 2,000 Gbp). More preferably, the size of sample DNA is approximately 3 Gbp, the amount of input DNA in PCR is 20 to 2,500 amol, and the amount of sequencing data as the number of read sequences or read pairs is 0.4 to 25,000 * 10⁶ (0.08 to 5,000 Gbp), preferably 0.8 to 12,500 * 10⁶ (0.16 to 2,500 Gbp), more preferably 1.6 to 6,250 * 10⁶ (0.32 to 1,250 Gbp), and even more preferably 3.2 to 5,000 * 10⁶ (0.64 to 1,000 Gbp). Even more preferably, the size of sample DNA is approximately 3 Gbp, the amount of input DNA in PCR is 39 to 1,250 amol, and the amount of sequencing data as the number of read sequences or read pairs is 0.78 to 12,500 * 10⁶ (0.156 to 2,500 Gbp), preferably 1.56 to 6,250 * 10⁶ (0.312 to 1,250 Gbp), more preferably 3.12 to 3,125 * 10⁶ (0.624 to 625 Gbp), and even more preferably 6.24 to 2,500 * 10⁶ (1.248 to 500 Gbp). In still another aspect, the size of sample DNA is approximately 3 Gbp, and the number of read sequences or read pairs per group of read sequences or read pairs is, as the mean among the groups, 1.05 to 30, preferably 1.1 to 20, even more preferably 1.2 to 10, and even more preferably 1.4 to 5.

Examples of sample DNA having a size of approximately 5 Mbp include the genome of Salmonella (approximately 4.86 Mbp). Preferred examples of Salmonella include an LT-2 strain, TA100 strain, TA98 strain, TA1535 strain, TA1538 strain, and TA1537 strain of S. typhimurium, which are applicable for the Ames test.

### (4. Applications of sequencing method)

Sequence data acquired in the method for sequencing DNA according to the present invention as defined in the claims are highly accurate sequence data excluding read errors in sequencing and errors due to oxidative modification of DNA or the like. Hence, this method for sequencing DNA may be applied to mutation analysis, though the application is not limited thereto. More specifically, for example, the method for sequencing DNA described herein may be applied to genotoxicity evaluation for a test substance, evaluation of other toxicity such as reproductive and developmental toxicity, evaluation of impacts of change over time, living environments, genetic factors, and so forth on genomic DNA, and quality evaluation for cultured cells, through mutation analysis for genomic DNA. In these applications, the sequencing method can be performed by using genomic DNA, which is a subject of mutation analysis, as sample DNA to acquire sequence data. Subsequently, mutation analysis is performed by using the sequence data acquired to detect a mutation in the genomic DNA of the subject of analysis.

Thus, the present invention as defined in the claims also provides a method for detecting a mutation in genomic DNA. This method comprises: performing the method for sequencing DNA according to the present invention as defined in the claims for genomic DNA in cells as sample DNA to generate sequence data; and comparing the sequence data with a reference sequence to detect a site of mismatch base between the sequence data and the reference sequence as a site of mutation.

The method for detecting a mutation in genomic DNA according to the present disclosure can be used for genotoxicity evaluation for a test substance. The genomic DNA can be genomic DNA from cells exposed to a test substance. The genomic DNA can comprise genomic DNA from cells exposed to a test substance (subject cells) and genomic DNA from cells not exposed to the test substance (control cells), and a mutation detected in the genomic DNA from the subject cells is compared with a mutation detected in the genomic DNA from the control cells. For example, a mutation detected only in the subject cells is identified as a mutation generated by exposure to the test substance. The cells to be used in the present method are not particularly limited, and examples thereof include microbial cells, animal cells, and plant cells. Preferred examples of animals include mammals, birds, silkworms, and nematodes, and examples of microbes include Escherichia such as Escherichia coli, Salmonella, and yeast. Preferred examples of cells to be used in the present method include cells of Salmonella and cells of Escherichia coli. Preferred examples of Salmonella are as described above. Preferred examples of Escherichia coli include K-12 strains, which are widely used for molecular biology study, and WP2 strains and WP2 uvrA strains, which are used in the Ames test. Other preferred examples of cells to be used in the present method include cells of mammals collected from the living body and cultured cells derived from mammals. Preferred examples of mammals include mice, rats, hamsters, Chinese hamsters, rabbits, and humans, among which mice and humans are preferred. Other examples of cells to be used in the present method may include cells of birds collected from the living body and cultured cells derived from birds. Preferred examples of birds include chickens, and examples of cultured cells derived from birds include DT40.

The test substance may be, for example, any substance the genotoxicity of which is desired to be evaluated. Examples thereof include substances suspected to have genotoxicity, substances for which the presence or absence of genotoxicity is desired to be determined, and substances for which the type of mutation to be induced is desired to be examined. The test substance may be a naturally-occurring substance, or a substance artificially synthesized, for example, through a chemical or biological method, or a compound, or a composition or mixture. Alternatively, the test substance may be an ultraviolet ray or radiation. Any means to expose cells to the test substance can be appropriately selected in accordance with the type of the test substance, and the means is not particularly limited. Examples thereof include a method of adding the test substance to a medium containing cells, and a method of subjecting cells under an atmosphere in which the test substance is present.

In another aspect, the method for detecting a mutation in genomic DNA described herein can be used for evaluation of impacts of change over time, living environments, genetic factors, and so forth on genomic DNA. Examples of change over time include growth, aging, senility, and subculture of cells or individual, examples of living environments include lifestyle such as dietary habit and exercise, and residence, and examples of genetic factors include sex, species, and deletion of a specific gene. A preferred example is the evaluation of impacts of change over time on genomic DNA, and genomic DNA from cells experienced change over time is used therefor. The genomic DNA may comprise genomic DNA from cells experienced change over time (subject cells) and genomic DNA from cells experienced change over time to a lesser extent (control cells), and a mutation detected in the genomic DNA from the subject cells is compared with a mutation detected in the genomic DNA from the control cells. Examples of cells experienced change over time to a lesser extent to be used as the control cells may include cells in which the degree of growth, aging, senility, or subculture is smaller than that of the subject cells (e.g., younger cells, cells without aging treatment, cells not subcultured or of smaller passage number). For example, a mutation detected only in the subject cells can be identified as a mutation generated by change over time. Examples of cells to be used may include cells of mammals collected from the living body and cultured cells derived from mammals. Preferred examples of mammals are as described above.

In another aspect, the method for detecting a mutation in genomic DNA according to the present disclosure can be used for quality evaluation for cultured cells. The genomic DNA to be used can be genomic DNA from cultured cells for which the presence or absence of mutation is desired to be examined. Examples of the cultured cells for which the presence or absence of mutation is desired to be examined include cells cultured for a certain period of time for which the tendency of mutation is desired to be checked. Preferably, the genomic DNA comprises genomic DNA from the cells for which the tendency of mutation is desired to be examined (subject cells) and genomic DNA from control cells. For the control cells, cultured cells of the same type whose genetic information is known (e.g., for which the presence or absence of mutation and the mutation type have been confirmed) are used. A mutation detected in the genomic DNA from the subject cells is compared with a mutation detected in the genomic DNA from the control cells. For example, a mutation detected only for the subject cells can be identified as a mutation generated during culturing.

Examples of mutation detected in the method for detecting a mutation in genomic DNA according to the present disclosure can include base-pair substitution mutation and short insertion or deletion mutation. Base-pair substitution mutation is mutation which changes a base pair to another base pair in base-pair information of DNA, and examples thereof include single base-pair substitution mutation and multiple base-pair substitution mutation, which includes substitution of two base pairs or three or more base pairs. Single base-pair substitution mutation is preferably detected. Short insertion or deletion mutation is mutation which causes insertion or deletion of a short base sequence into or from a DNA sequence, where the length of bases inserted or deleted is preferably 10 bp or smaller, and more preferably 1 to 5 bp.

In order to investigate the tendency of mutation in the subject whole genomic DNA for analysis, priority is given to analysis of the tendency of mutation in a wider genomic region through analysis of mutation patterns by detecting mutations in a wider region of genome, rather than strict identification of the presence or absence of a mutation at a specific site of genome.

A preferred procedure in detecting patterns of single base-pair substitution mutation in subject genomic DNA for analysis will be described in the following. In detecting base-pair substitution mutation, sequence data acquired in the method for sequencing DNA according to the present disclosure are compared with a reference sequence, and a site of mismatch base between the sequence data and the reference sequence is detected as a site of mutation. The site detected is acquired as a site of mutation with base-pair substitution mutation. A part or all of the sequence data acquired may be used for comparison with the reference sequence in accordance with the purpose of mutation analysis.

Subsequently, mutations are classified by mutation patterns of bases on the basis of the type of a base at a detected site of mutation and the type of a base before mutation. Further, frequency of appearance can be determined for each of the mutation patterns of bases. The procedure can be performed, for example, by using programs written in a programming language such as Python.

In a more specific example, bases contained in sequence data are classified into the following (i) to (iv).
(i) a base located at a position where the base on the reference sequence is A,
(ii) a base located at a position where the base on the reference sequence is T,
(iii) a base located at a position where the base on the reference sequence is G, and
(iv) a base located at a position where the base on the reference sequence is C.
The bases (i) and (ii) are bases present at sites where the base pair on the reference sequence is AT. The bases (iii) and (iv) are bases present at sites where the base pair on the reference sequence is GC. Among these bases, a mismatch base with respect to the reference sequence (i.e., base with base-pair substitution mutation) is detected. Subsequently, base pairs before and after mutation are determined for each of the detected sites of mutation on the basis of information on the reference sequence and sequence data. From these data, mutations can be classified into six mutation patterns of base pairs in total, namely, three patterns, [AT → TA, AT → CG, and AT → GC], for the case that the base pair before mutation is AT and three patterns, [GC → TA, GC → CG, and GC → AT], for the case that the base pair before mutation is GC. Further, frequency of appearance can be determined for each mutation pattern on the basis of the total number of mutations belonging to each mutation pattern and the total number of bases analyzed. For example, on the basis of the total number of bases analyzed for each of the base pairs AT and GC, frequency of appearance can be calculated for three mutation patterns for each of the base pairs.

Further, the present disclosure allows analysis of multi-base-pair substitution mutation. Examples of multi-base-pair substitution mutation include two-base-pair substitution mutation and three-base-pair substitution mutation. In analysis of multi-base-pair substitution mutation, for example, mutation patterns are classified on the basis of a base sequence before mutation (e.g., 4 * 4 = 16 patterns for two-base-pair substitution mutation), and then frequency of appearance can be determined for each mutation pattern on the basis of the total number of mutations belonging to each mutation pattern and the total number of mutations analyzed.

Further, the present disclosure allows sequence context analysis of single-base-pair substitution mutation. In this analysis, single-base-pair substitution mutations are detected in the above procedure, and a sequence including a base before mutation and the adjacent bases in the upstream and downstream of the base before mutation (what is called context) is then determined for each of the detected mutations on the basis of a reference sequence. Subsequently, each mutation is typed by mutation patterns of base pairs and the context. Specifically, the mutations detected are classified into six mutation patterns of base pairs, [AT → TA, AT → CG, AT → GC, GC → TA, GC → CG, and GC → AT], by using the above-described procedure. Meanwhile, each mutation detected is classified by contexts. For example, contexts having a length of three bases including adjacent one base in each side of a site of mutation are classified into 4 * 4 = 16 groups, [e.g., for mutations from C, ACA, ACC, ACG, ACT, CCA, CCC, CCG, CCT, GCA, GCC, GCG, GCT, TCA, TCC, TCG, and TCT]. As a result, each mutation is classified into 96 (4 * 6 * 4) types in total by mutation patterns of base pairs and contexts. Analysis with longer contexts is also applicable. For example, each mutation is classified into 256 (4 * 4 * 4 * 4) groups by contexts having a length of five bases including adjacent two bases in each side of a site of mutation, and through the classification and the six base pair patterns, each mutation is ultimately classified into 1536 (4 * 4 * 6 * 4 * 4) types in total. Further, each mutation is classified into 4²ⁿ groups by contexts having a length of (2n + 1) bases including n adjacent bases in each side of a site of mutation, and through the classification and the six base pair patterns, each mutation is ultimately classified into (4²ⁿ * 6) types in total. Subsequently, frequency of mutation can be determined for each of the mutation types on the basis of the total number of mutations belonging to each mutation type and the total number of bases analyzed.

Next, a preferred procedure in detecting short insertion or deletion mutation in genomic DNA as a target of analysis will be described. In detecting short insertion or deletion mutation, each sequence data is compared with a reference sequence to detect a site where a base is inserted into or deleted from the reference sequence in the sequence data. In comparing with the reference sequence, sequence data acquired may be partly used or totally used. The site of insertion or deletion to be detected is a site where the length of inserted or deleted bases is preferably 10 bp or smaller, and more preferably 1 to 5 bp, though the length is not limited thereto. Sites detected are acquired as sites of mutation with insertion or deletion mutation.

For each of the mutations acquired, the type of mutation (insertion mutation or deletion mutation), the base length of the site of insertion or deletion, or the type of a base(s) inserted or deleted can be determined. The procedure to detect a site of insertion or deletion with a specific base length can be performed by using the above-described programs written in a programming language such as Python. Further, the type of a base(s) inserted or deleted can be identified by comparing sequence data with a reference sequence. Through these operations, the base length of each site of insertion or deletion in sequence data or the type of a base(s) at each site of insertion or deletion can be determined. In addition, frequency of insertion or deletion may be determined for each base length and/or each type of base. For example, insertion or deletion mutations acquired for read sequences can be classified by base length to determine frequency for each base length. For example, bases inserted or deleted can be classified by type (A, T, G, and C) to determine frequency for each type. Further, mutations can be more finely classified by combination of the classifications by base length and type of base to determine frequency for each of the combined classifications.

### Examples

Hereinafter, Examples will be shown to more specifically describe the present invention as defined in the appended claims. Examples which do not fall under the scope of the claims are for reference purposes only.

In the following Example 1, an algorithm was constructed which enables highly accurate sequencing based only on sequence information on sample DNA, without using a label to individually identify DNA fragments, through use of information on a plurality of read sequences for each one fragment of sample DNA.

In addition, optimum conditions for the sequencing method using the above algorithm were studied in Examples 2 to 5. As described above, PCR conditions in library preparation, amount of sequencing, and the size of genome of a target for analysis were expected to be primarily important as factors which can affect the efficiency and accuracy of the sequencing. In view of this, the inventors studied, through adjusting those factors, on optimum conditions which achieve an accuracy needed to figure out the tendency of mutations caused by chemicals or the like in the whole genome and provide maximized efficiency, for the sequencing method using the plurality of read sequences containing information on complementary strands of sample DNA. In Examples, the sequencer HiSeq from Illumina, Inc., which is the most widely used currently, was used.

### Example. 1

Sequencing algorithm using plurality of read sequences

### 1) Sequencing algorithm using information on complementary strands of DNA

In this algorithm, read sequences (read pairs) expected to be derived from a PCR product of the same DNA fragment are collected, without using a label to individually identify DNA fragments (e.g., a tag sequence specific to a fragment molecule), and then a consensus read sequence (a consensus read sequence considering complementary strands) is generated among read sequences expected to be derived from two complementary strands (hereinafter, referred to as strand A and strand B), which is acquired as sequence data. Acquired consensus read sequences considering complementary strands can be used for mutation analysis of a target DNA or the like.

A basic flow to acquire such consensus read sequences considering complementary strands will be described in the following.
i) Library pools to be used in the present Example each contain PCR products derived from both of two complementary strands of each fragment of sample DNA. Hence, when such a library pool is subjected to sequencing, read 1 and read 2 can be generated for each of the two complementary strands.
ii) To identify read sequences for strands complementary to each other, adapter sequences including an unsymmetrical label DNA sequence at a terminal are linked to each terminal of each fragment of sample DNA before PCR. Identical pairs of adapter sequences are added to the both terminals of all the fragments of sample DNA. Thus, identical label sequences are added to the 5'-terminal of strand A and the 5'-terminal of strand B, and other identical label sequences are added to the 3'-terminal of strand A and the 3'-terminal of strand B, and, meanwhile, different label sequences are added to the 5'-terminal and the 3'-terminal in one strand for all the fragments of sample DNA. Subsequently, PCR with primers which specifically bind to the adapter sequences is performed to produce PCR products including the adapter sequences, and the PCR products are used as a library pool for sequencing. Each of the amplified products is bound onto a flow cell, which is used in sequencing described later, through annealing of the adapter sequence included in the amplified product with an oligo DNA fragment on the flow cell. Typically, only amplified products derived from one strand are allowed to be present on the flow cell and subjected to sequencing reaction.
iii) Sequencing is performed for the library pool obtained. In sequencing, a pair of two read sequences (read 1, read 2) is acquired for each amplified fragment (derived from a fragment of sample DNA) contained in each PCR product in the library pool. Here, the read sequence containing sequence information on reading out of the sequence of one strand of the amplified fragment from the 5'-terminal side to the 3'-terminal side is read 1 (R1), and the read sequence containing sequence information on reading out of the sequence of the same strand from the 3'-terminal side to the 5'-terminal side is read 2 (R2). Schematic Diagram 1 shows a conceptual diagram illustrating location of read pairs derived from two complementary strands of a fragment of sample DNA in a reference sequence when the read pairs are mapped on the reference sequence. For reference, two complementary strands of a fragment of sample DNA from which the read pairs are derived are illustrated in Schematic Diagram 1. A region between the beginning of read 1 and the beginning of read 2 in the reference sequence is the same among read pairs derived from strands complementary to each other. Therefore, read pairs expected to be derived from the same fragment of sample DNA can be collected on the basis of the position of each read pair mapped on the reference sequence.

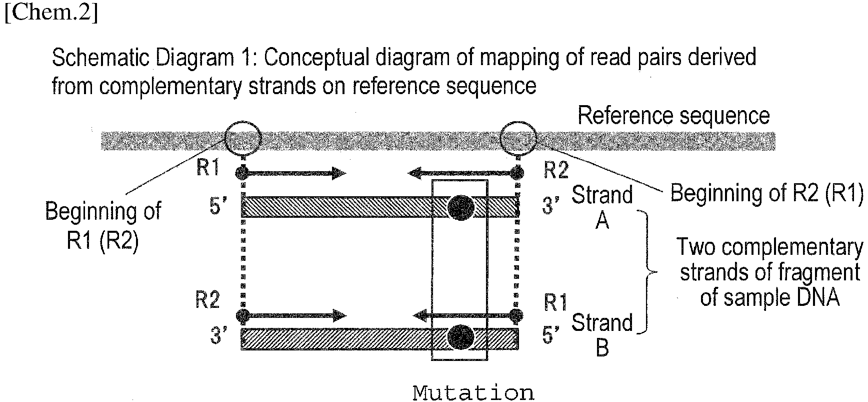

Terms in Examples below are defined as follows: "estimated fragment" refers to a region from the beginning of read 1 (read 2) to the beginning of read 2 (read 1) mapped on a reference sequence, in other words, a region between the beginning of read 1 and the beginning of read 2 when read pairs (read 1, read 2) are mapped on the reference sequence; and "group" for the estimated fragment refers to a group of read pairs to give a common estimated fragment (Schematic Diagram 2).

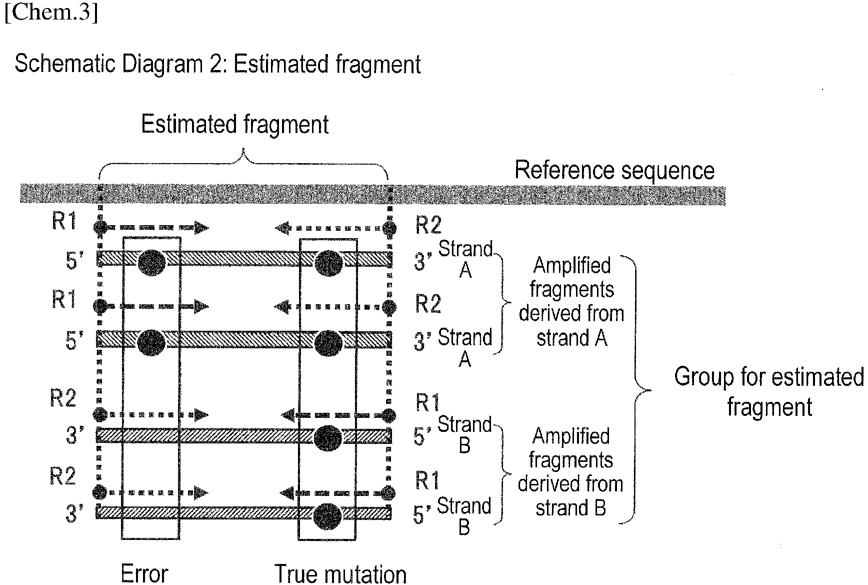

iv) Subsequently, from the group for an estimated fragment, combination of the read pairs from one and the other of two strands complementary to each other is acquired as a set of read pairs.

Amplified fragments obtained from a fragment of sample DNA may possess mutations originally included in the fragment of sample DNA in both strands, and may additionally include a base variation only in one strand due to base modification of the fragment of sample DNA or the like. Such a case is illustrated in Schematic Diagrams 1 and 2. The fragment of sample DNA shown in Schematic Diagram 1 possesses one base substitution due to mutation in each strand. On the other hand, the amplified fragments derived from the fragment of sample DNA shown in Schematic Diagram 2 possess a base substitution due to mutation (true mutation) in both strands, and additionally include a base substitution generated in the course of sample preparation (error) only in one strand. These true mutation and error have been read out into read 1 and read 2 of each read pair. Hence, the true mutation fixed in both strands can be distinguished from the error generated only in one strand and the true mutation can be extracted, through use of sequence information from complementary strands contained in the set of read pairs.

In the present algorithm, a consensus read sequence considering complementary strands is generated from each set of collected read pairs. In generating consensus read sequences considering complementary strands, read pairs to give a common estimated fragment are first collected, and the collected read pairs are classified into read pairs derived from strand A and read pairs derived from strand B. Subsequently, combination of one or more read pairs derived from strand A and one or more read pairs derived from strand B is acquired as a set of read pairs, and a consensus read sequence considering complementary strands is generated by using the set of read pairs. The number of read pairs derived from strand A or strand B is not particularly limited, and it is only required to include at least one or more read pairs derived from strand A and at least one or more read pairs derived from strand B. Even in the case that the number of read pairs derived from strand A is two and the number of read pairs derived from strand B is two, for example, or in the case that the number of read pairs derived from strand A is three and the number of read pairs derived from strand B is one, a consensus read sequence considering complementary strands is generated by building consensus among them.

Schematic Diagram 3 below shows, as an example, a more detail procedure from making collections of read pairs to generating consensus read sequences considering complementary strands. In the procedure in Schematic Diagram 3, read pairs derived from each complementary strand are first mapped on a reference sequence (1). Then, a group of read pairs whose left ends (ends closest to the 5'-terminal in the reference sequence) are present at the same position in the reference sequence is acquired as a first collection (2). Subsequently, read pairs whose right ends (ends closest to the 3'-terminal in the reference sequence) are present at the same position in the reference sequence are separated from the first collection and acquired as a second collection (3). This second collection is a collection of read pairs to give a common estimated fragment. The second collection is then classified into a group derived from strand A (group F) and a group derived from strand B (group R) (4). Here, the group derived from strand A and the group derived from strand B can be distinguished on the basis of information on label sequences acquired in sequencing. In the present Example, sequencing reaction was performed by using a flow cell which recognizes the label sequence in each adapter sequence added to each fragment of sample DNA and allows it to bind to the flow cell. After amplification of fragments in the flow cell, the label sequence in the adapter sequence added in the 5'-terminal side was specifically cut to unify the direction of sequencing for read 1 and read 2 for each amplified fragment, and read pairs were classified into group F and group R on the basis of information on the label sequence. Group F and group R are each a collection of read pairs derived from either one of two complementary strands constituting a DNA fragment. Hence, a consensus read sequence considering complementary strands can be generated by building consensus between group F and group R (5).

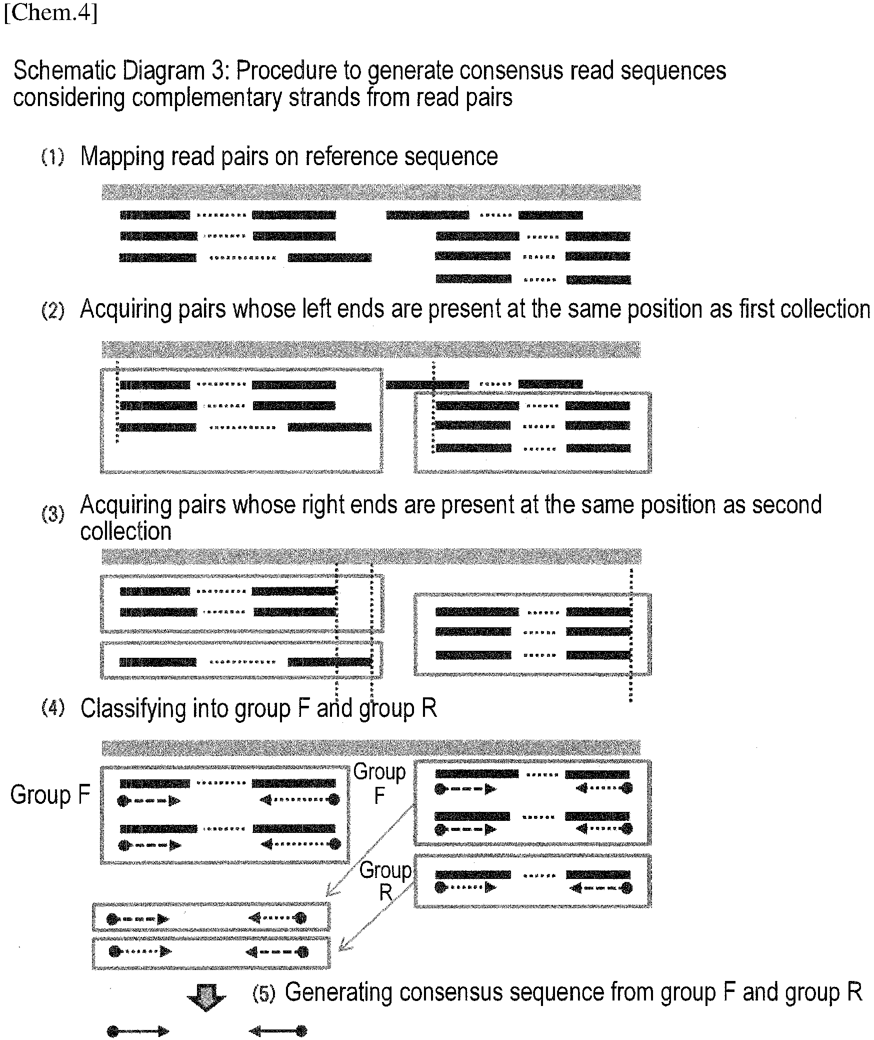

By generating consensus read sequences considering complementary strands, mutations present in both strands in common can be acquired as true mutations while mutations generated only in one strand are excluded as errors. This procedure, which excludes analysis errors from true mutations with use of information on complementary strands, theoretically enables enhancement of sequencing accuracy.

### 2) Mutation analysis based on consensus read sequences considering complementary strands

Consensus read sequences considering complementary strands acquired in 1) can be used for various analyses. As a representative example, mutation analysis for genomic DNA using consensus read sequences considering complementary strands will be described in the following.

Fundamentally, genomic DNA of a target for analysis is first subjected to sequencing by using the procedure of 1) to generate consensus read sequences considering complementary strands. Subsequently, mutations in the target genome are detected by using the consensus read sequences considering complementary strands through a common procedure. For example, mutations in the target genome can be detected by re-mapping the consensus read sequences considering complementary strands on the reference sequence. Mutation analyses for actual data in the following Examples were performed in accordance with a flow illustrated in Schematic Diagram 4. In analyses, the software Cutadapt, the software Bowtie2, the software Samtools, and programs written in the programming language Python were used. A detail procedure was according to those described in PCT/JP2017/005700 to detect mutated bases from re-mapped consensus read sequences considering complementary strands on a reference sequence. The detail procedure for detection will be described later in Examples 2 and 3.

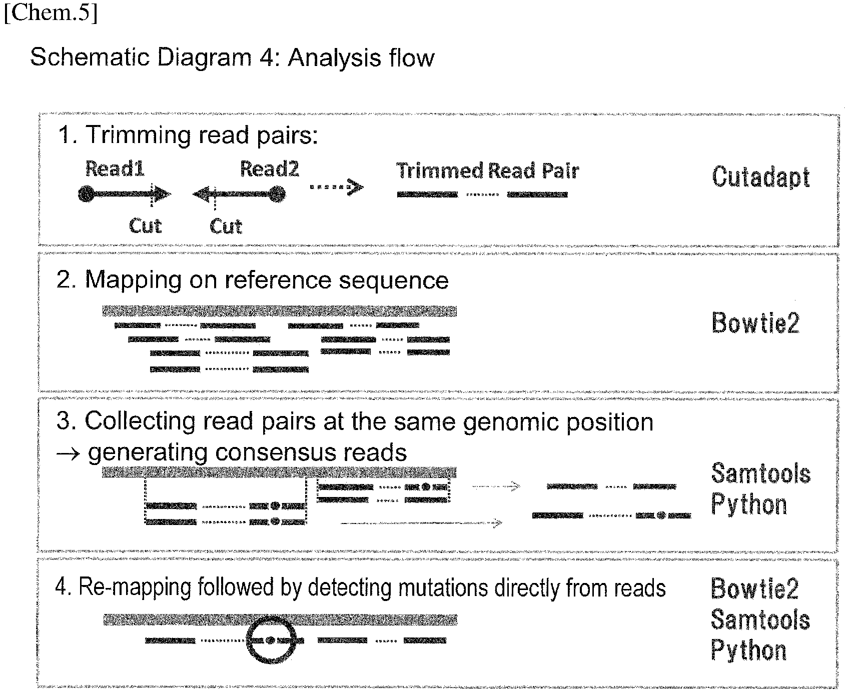

### Example. 2

Search for optimum sequencing conditions using mutagen-exposed DNA sample In the present Example, optimum conditions for sequencing with the algorithm described in Example 1 were searched for in order to acquire sequence data for genomic mutation analysis. Genomic DNA from TA100 strain of Salmonella typhimurium (S. typhimurium) LT-2 (hereinafter, also referred to as "TA100 strain", simply) exposed to ethylnitrosourea (ENU, CASRN.759-73-9), a representative mutagen, was used as sample DNA.

### 1) Preparation of genomic DNA sample from TA100 strain

Exposure of cells to ENU was performed in accordance with the pre-incubation method of the Ames test (K. Mortelmans et al. Mutat. Res. - Fundam. Mol. Mech. Mutagen. 455:29-60, 2000). TA100 strain was seeded in 2 mL of Nutrient Broth No. 2 (produced by Oxoid Limited), and subjected to shaking culture at 37°C and 180 rpm for 4 hours to afford pre-culture solution with an O.D.660 value of 1.0 or higher. ENU (produced by Sigma-Aldrich Co. LLC.) was diluted with dimethylsulfoxide (DMSO; produced by Wako Pure Chemical Industries, Ltd.). Into test tubes, 100 µL of the ENU solution, 500 µL of 0.1 M phosphate buffer, and 100 µL of the pre-culture solution were added (amount of ENU: 62.5, 125, 250, 500, and 1,000 µg/tube), and the resultants were subjected to shaking culture at 100 rpm in a water bath at 37°C for 20 minutes (ENU group). For a control group, 100 µL of solvent (DMSO) was added instead of the ENU solution. After shaking culture for 20 minutes, the test tubes each containing a culture solution were taken out of the water bath, and 50 µL of each culture solution was added to 2 mL of Nutrient Broth solution aliquoted in advance, and the resultant was further cultured in an incubator at 37°C and 180 rpm for 14 hours, from which the bacterial suspensions were then collected and centrifuged at 7,500 rpm for 5 minutes, and the supernatants were removed to collect the bacterial cells.

For the Ames test, a bacterial suspension exposed to ENU by using the pre-incubation method was produced under the above-described conditions, and 2 mL of top agar (containing 1% NaCl, 1% agar, 0.05 mM histidine, and 0.05 mM biotine) warmed to 45°C was added thereto and the resultant was suspended by using a Vortex, and poured onto a minimal glucose agar medium (Tesmedia (R) AN; produced by Oriental Yeast Co., Ltd.) to form a layer. The resulting plate was subjected to culturing at 37°C for 48 hours, and the number of colonies observed was counted.

### 2) Collection of total DNA and concentration measurement

From the bacterial cells obtained in 1) (control group and ENU group), total DNA was collected by using a DNeasy Blood & Tissue Kit (produced by QIAGEN) in accordance with the recommended protocol. The double-stranded DNA concentration of each DNA sample obtained was measured by using a Qubit3.0 Fluorometer (produced by Thermo Fisher Scientific) with a Qubit (TM) dsDNA BR Assay Kit attached thereto.

### 3) Preparation of libraries for sequencing

From each DNA sample (control group and ENU group) after being subjected to concentration measurement in 2), a plurality of samples each of which was to give 100 ng of DNA were prepared, and each sample was fragmented into pieces having a length of approximately 350 bp on average by using a DNA Shearing System ME220 (produced by Covaris Inc.) in accordance with the recommended protocol. The fragmented DNA obtained was subjected to library preparation by using a TruSeq Nano DNA Library Prep Kit (produced by Illumina, Inc., hereinafter abbreviated as TruSeq) in accordance with the recommended protocol.

In library preparation, the DNA fragments obtained were each labeled with adapter sequences each including a label sequence at a terminal, as described in Example 1. The resultant was subjected to PCR under different conditions listed in Table 1 to prepare libraries for sequencing, thus preparing labeled libraries for sequencing with different conditions. Adapter sequences attached to the TruSeq from Illumina, Inc. were used. As shown in Table 1, adapter sequences to be used were changed for different libraries, where two different adapters distinguished by different index sequences (1^{st} Index and 2^{nd} Index) were used for each library. Thus, one of two adapters was bound to each fragment in each library. Specifically, an adapter of 1^{st} Index or 2^{nd} Index was added to a solution for library preparation for each sample (control group and ENU group) as shown in Table 1, and the resulting two solutions for library preparation were separately subjected to ligation reaction. Each of the resulting ligation reaction solutions was purified in accordance with the recommended protocol, and then subjected to concentration measurement for ligation products by using a High Sensitivity D5000 Kit in an Agilent 4200 TapeStation (produced by Agilent Technologies). On the basis of concentrations measured, each sample was serially diluted with Resuspension Buffer attached to the TruSeq as shown in Table 1, and 12.5 µL of a diluted solution of the ligation product with the adapter of 1^{st} Index and 12.5 µL of a diluted solution of the ligation product with the adapter of 2^{nd} Index were mixed together to give 25 µL of a mixed solution. The mixed solution obtained was subjected to purification through PCR in accordance with the recommended protocol. To adjust difference in amount of input DNA in PCR, each sample was amplified through PCR cycles the number of which is shown in Table 1 to adjust the amount of DNA in the library.

**[Table 1]**

| Library No. | 1 | 2 | 3 | 4 | 5 | 6 | 7 | 8 |
|---|---|---|---|---|---|---|---|---|
| DNA concentration (pmol/L) | 800 | 400 | 200 | 100 | 50 | 25 | 12.5 | 6.25 |
| PCR input (amol) | 20000 | 10000 | 5000 | 2500 | 1250 | 625 | 313 | 156 |
| PCR input (amol)/Mbp (genome) | 4000 | 2000 | 1000 | 500 | 250 | 125 | 62.5 | 31.3 |
| 1^{st}Index* | 2 | 4 | 5 | 6 | 7 | 12 | 13 | 10 |
| 2^{nd} Index* | 15 | 16 | 18 | 19 | 1 | 3 | 8 | 9 |
| Number of PCR cycles | 7 | 8 | 9 | 10 | 11 | 12 | 13 | 14 |

| Library No. | 9 | 10 | 11 | 12 | 13 | 14 |
|---|---|---|---|---|---|---|
| DNA concentration (pmol/L) | 6.25 | 3.13 | 1.56 | 0.78 | 0.39 | 0.20 |
| PCR input (amol) | 156 | 78 | 39 | 20 | 10 | 5 |
| PCR input (amol)/Mbp (genome) | 31.3 | 15.6 | 7.8 | 3.9 | 2 | 1 |
| 1^{st} Index* | 2 | 4 | 5 | 6 | 7 | 12 |
| 2^{nd} Index* | 13 | 14 | 15 | 16 | 18 | 19 |
| Number of PCR cycles | 14 | 15 | 16 | 17 | 18 | 19 |

| | | | | | | |
|---|---|---|---|---|---|---|
| *: Identification number of Index | | | | | | |

### 4) Sequencing

Each of the libraries prepared in 3) was subjected to sequencing by using a HiSeq 2500 (produced by Illumina, Inc.), where the read length was set to 2 * 125 bp for Library Nos. 1 to 8 shown in Table 1, and to 2 * 100 bp for Library Nos. 9 to 14. Sequencing data of approximately 10 Gbp were acquired on average per library.

### 5) Edit of read sequences, extraction of information on complementary strands, and mutation analysis

Raw read sequences acquired through sequencing were edited and subjected to mutation analysis in accordance with the analysis flow illustrated above in Schematic Diagram 4. First, adapter sequences and bases such as those of low quality were trimmed from raw read sequences to generate read sequence pairs by using the software Cutadapt. Subsequently, for the Fastq files containing the read pairs acquired after trimming, Fastq files derived from the same library were combined into one Fastq file on the basis of index information for adapter sequences. The index information for adapter sequences is information liked to read sequence information, but is not information contained in each read sequence. Meanwhile, sequencing data of approximately 2 Gbp (10 * 10⁶ (10 M) read pairs) were analyzed for analysis with a smaller amount of sequencing data. Specifically, from each of the beginning and end of the Fastq file containing the sequencing data of approximately 10 Gbp (10 Gbp Fastq file), 5 * 10⁶ read pairs (10 * 10⁶, 10 M read pairs in total) were extracted and combined into one file to generate a Fastq file for analysis for approximately 2 Gbp (2 Gbp Fastq file). Thereafter, each of these Fastq files (10 Gbp and 2 Gbp) was subjected to mapping on a reference sequence by using the software Bowtie2 to provide a file in Sam format. The read of each file in Sam format was sorted by using the software Samtools, and sets of read pairs derived from two complementary strands of a common estimated fragment were extracted to generate consensus read sequences considering complementary strands from the sets of read pairs, by using programs written in the programming language Python in accordance with the algorithm shown in Example 1 (see Schematic Diagram 3). The acquired consensus read sequences considering complementary strands were output as a new file in Fastq format. The acquired consensus read sequences considering complementary strands were re-mapped on the reference sequence by using the software Bowtie2, and mutation analysis was performed by using the software Samtools and programs written in the programming language Python. The reference sequence used for mapping by using the software Bowtie2 was a genome sequence from TA100 strain of S. typhimurium described in PCT/ JP2017/005700.

### 6) Calculation of distribution of number of read pairs

For each of the 10 Gbp Fastq file and 2 Gbp Fastq file from each library, which had been generated in 5), the number of read pairs per group for an estimated fragment was counted on the basis of the mapping results, and the number of groups including an equal number of read pairs was counted and compiled to determine the distribution of the number of read pairs per group. From this distribution, the mean number of read pairs/group was calculated for each library.

### 7) Calculation of overlap rates

From each of the 10 Gbp Fastq file and 2 Gbp Fastq file from each library, which had been acquired in 5), groups including two or more read pairs were extracted from groups for estimated fragments on the basis of the mapping results. For the groups extracted, the fraction of groups including read pairs derived from different DNA fragments (read pairs not derived from two complementary strands of the same fragment) (overlap rate) was calculated on the basis of index information for adapter sequences for each read pair. Specifically, each read pair was checked for the index sequence (1^{st} Index or 2^{nd} Index), and the number of groups including both read pair with 1^{st} Index and read pair with 2^{nd} Index and the ratio of it to the total number of the extracted groups including two or more read pairs (overlap rate) were calculated (Schematic Diagram 5). Overlap rate (%) = (number of groups including both of read pair with 1st Index and read pair with 2nd Index)/(number of groups including two or more read pairs) * 100

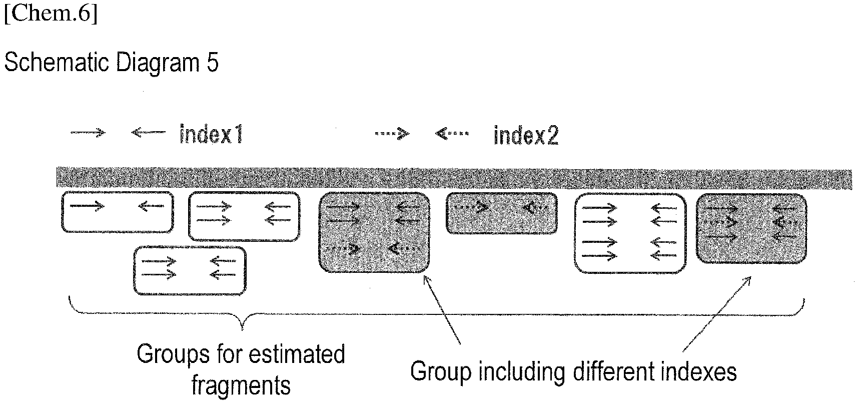

### 8) Calculation of mutation frequency and data efficiency

After consensus read sequences considering complementary strands were mapped on the reference sequence in 5), mutated bases were detected through a procedure described in PCT/JP2017/005700. Specifically, for each library, all bases targeted in analysis in all of the consensus read sequences considering complementary strands mapped on the reference sequence were classified by the corresponding base in the reference sequence into four groups by using a program written in Python. Subsequently, the total number of bases was counted and mutated bases as compared with the reference sequence were detected for each group. By comparing mutated bases detected and bases in the reference sequence, mutation patterns (AT → TA, AT → CG, AT → GC, and GC → TA, GC → CG, GC → AT) and the mutation frequency for each mutation pattern per 10⁶ bps of each AT base pairs and GC base pairs in the bases targeted in the analysis were calculated, for each of the control group and the ENU group. In addition, data efficiency was calculated for each library from the total amount (bp) of the target bases in the consensus read sequences for each of the libraries used in the mutation analysis and the amount (bp) of sequencing data made in the sequencing of each library. Data efficiency (%) = (total amount of bases targeted in analysis in consensus read sequences considering complementary strands)/(amount of sequencing data) * 100

### 9) Results and discussion

### I) Number of revertants in Ames test

Table 2 shows the number of revertant colonies after exposure to ENU. The data shown include measurements for three plates and mean values thereof. The number of revertant colonies was found to increase through exposure to ENU, and this result demonstrated that mutations had been introduced into the genome of TA100 strain through exposure to ENU.

**[Table 2]**

| Number of revertant colonies in Ames test | | | | |
|---|---|---|---|---|
| ENU concentration (µg/plate) | Number of revertants | | | |
| | 1 | 2 | 3 | Mean |
| 0 (DMSO) | 116 | 129 | 114 | 119.7 |
| 62.5 | 220 | 192 | 210 | 207.3 |
| 125 | 430 | 391 | 323 | 381.3 |
| 250 | 1482 | 1359 | 1326 | 1389.0 |
| 500 | 4095 | 4006 | 3751 | 3950.7 |
| 1000 | 1659 | 3206 | 2737 | 2534.0 |

### II) Impact of amount of input DNA on data efficiency and distribution of number of read pairs in sequencing

### i) 10 Gbp sequencing data

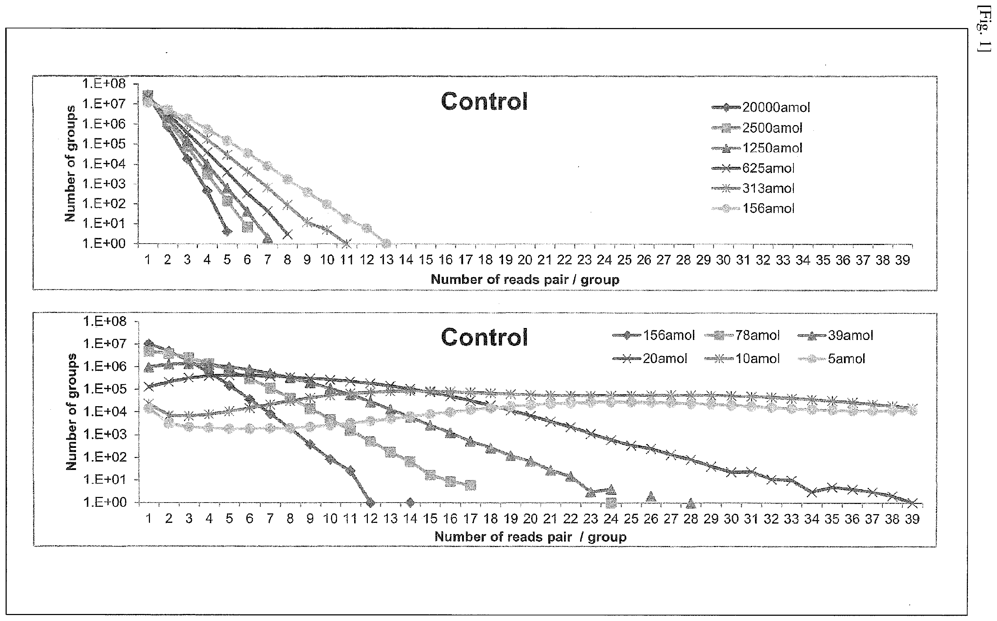

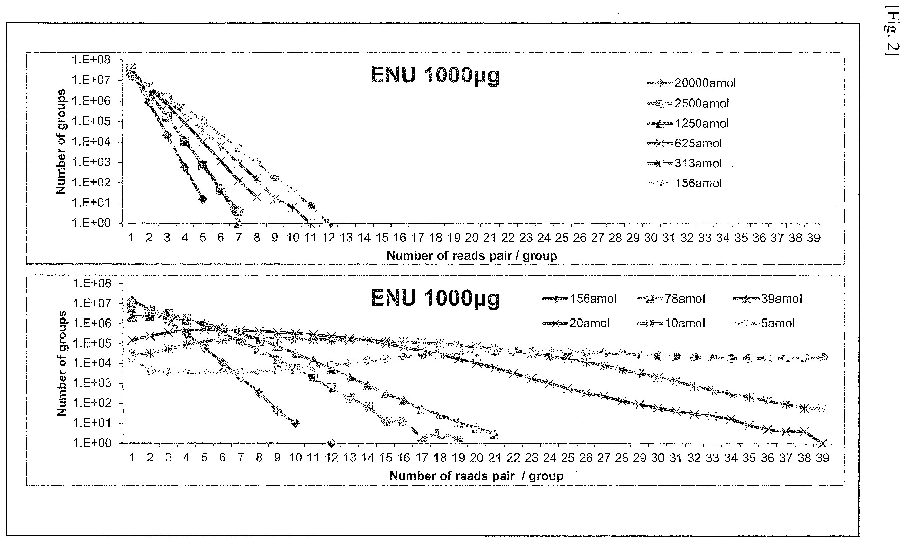

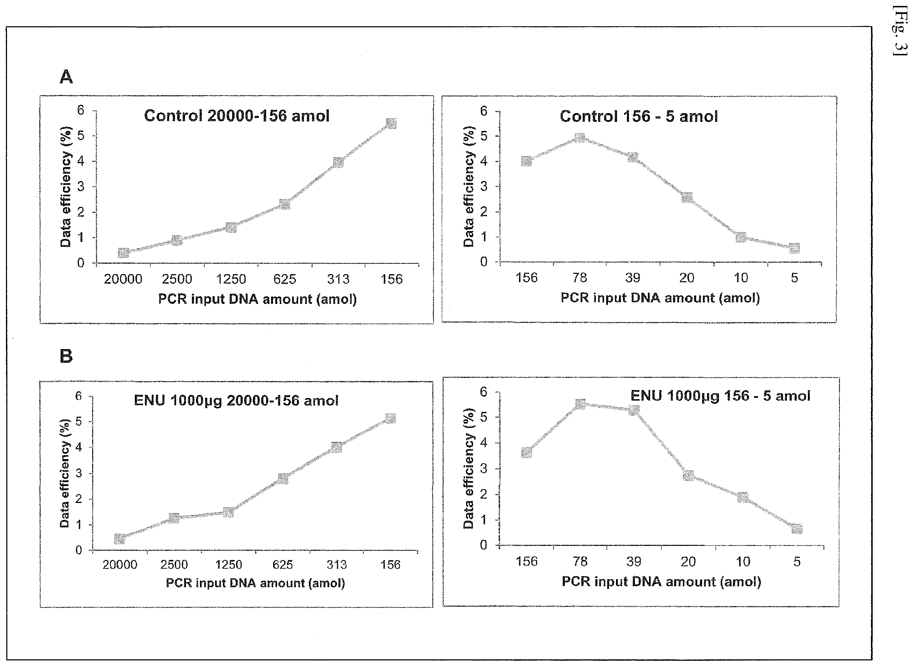

Figures 1 and 2 each show the distribution of the number of read pairs per group for an estimated fragment in a 10 Gbp Fastq file, which had been calculated in 6). Figures 1 and 2 show data for the control (DMSO-exposed) group and data for the ENU (ENU-exposed) group, respectively, for libraries (Library Nos. 1 and 4 to 14) with different amounts of input DNA in PCR. For both of the control group and the ENU group, it was found that the number of read pairs per group tended to increase as the amount of input DNA in PCR was reduced. Figure 3 shows data efficiency calculated in 8) for different amounts of input DNA in PCR. In the result for the control group, when libraries with the amount of input DNA of 20,000 to 156 amol (approximately 4,000 to 31.3 amol/Mbp (genome)) were subjected to sequencing, the library with 156 amol provided the best efficiency, supposing that further reduction of amount of input DNA would further enhance the efficiency. In view of this, libraries with the amount of input DNA of 156 to 5 amol (approximately 31.3 to 1 amol/Mbp (genome)) were subjected to sequencing, and as a result the library with 78 amol provided the best efficiency, indicating that 78 amol (approximately 15.6 amol/Mbp (genome)) is an amount of input DNA that provides the best data efficiency.

### ii) 2 Gbp sequencing data

Analysis was performed for the 2 Gbp Fastq file in the same manner as in i).

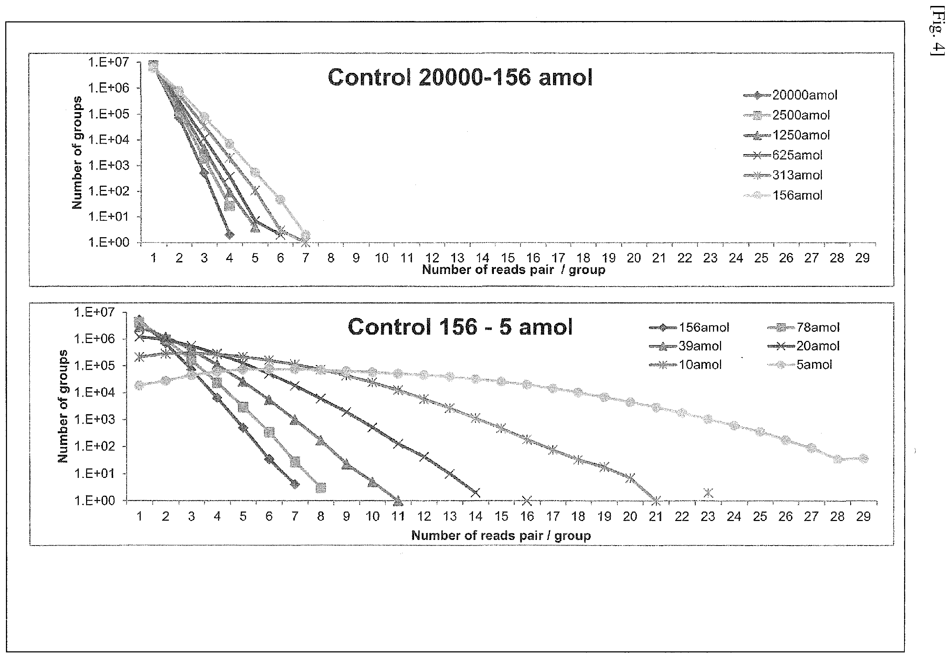

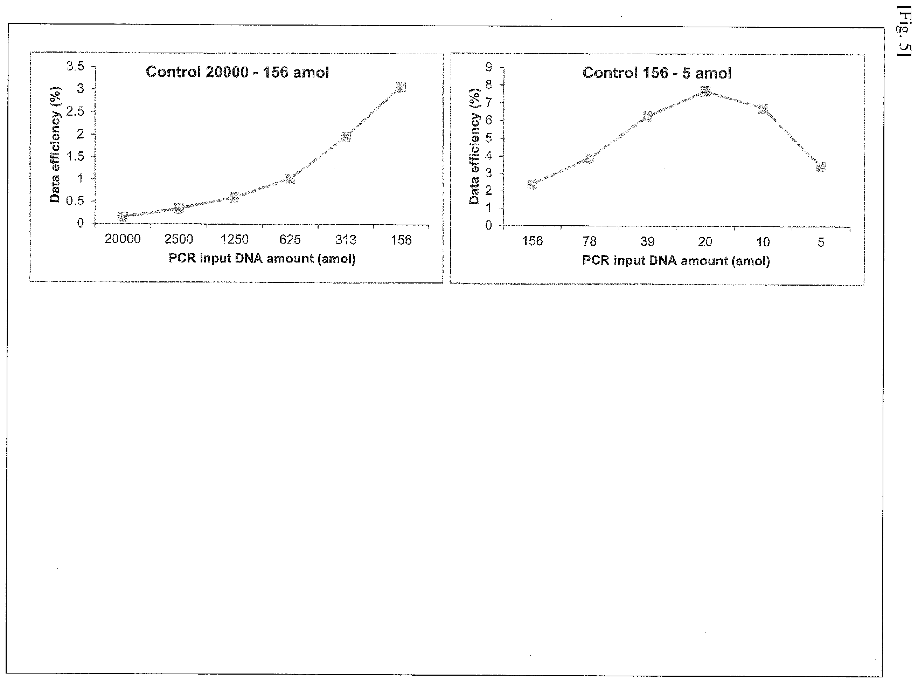

Analysis for the 2 Gbp sequencing data was performed only for the control group. As with the case of 10 Gbp data, it was found that the number of read pairs per group tended to increase as the amount of input DNA in PCR was reduced (Figure 4). In contrast to the case of 10 Gbp data, data efficiency was maximized when the amount of input DNA was 20 amol (Figure 5).

### iii) Relation between data efficiency and number of read pairs

If the number of read pairs included in each group is smaller on average, the number of groups available for generating consensus read sequences considering complementary strands may be smaller, leading to lowered data efficiency. If the number of read pairs included in each group is excessively large on average, on the other hand, the number of reads to be used for generating one consensus read sequence considering complementary strands may be larger, leading to rather lowered efficiency. Data efficiency and the mean number of read pairs per group for an estimated fragment were examined for the 10 Gbp sequencing data and 2 Gbp sequencing data for different amounts of input DNA. The results are shown in Table 3. For both of the 10 Gbp sequencing data and 2 Gbp sequencing data, the mean number of read pairs basically increased as the amount of input DNA was reduced. For the 10 Gbp sequencing data, the mean number of read pairs with 78 amol, which provided the best efficiency, was 2.35 for the control group, and 2.32 for the ENU group. For the 2 Gbp sequencing data, the mean number of read pairs with 20 amol, which provided the best efficiency, was 2.21, which was a value close to the value for 10 Gbp. These results demonstrate that optimum data efficiency is achieved if the number of read pairs included in each group for an estimated fragment is approximately 2 on average.

**[Table 3]**

| 10 Gbp sequencing data | | | | | | | | | | | | | |
|---|---|---|---|---|---|---|---|---|---|---|---|---|---|
| Library No. | | 1 | 4 | 5 | 6 | 7 | 8 | 9 | 10 | 11 | 12 | 13 | 14 |
| PCR input DNA amount (amol) | | 20000 | 2500 | 1250 | 625 | 313 | 156 | 156 | 78 | 39 | 20 | 10 | 5 |
| Control | Data efficiency (%) | 0.40 | 0.89 | 1.42 | 2.32 | 3.96 | 5.50 | 4.01 | 4.95 | 4.17 | 2.57 | 0.99 | 0.56 |
| | Mean Number of read pairs/group | 1.03 | 1.06 | 1.09 | 1.16 | 1.32 | 1.60 | 1.61 | 2.35 | 4.12 | 7.38 | 21.04 | 35.28 |
| ENU | Data efficiency (%) | 0.44 | 1.25 | 1.48 | 2.81 | 4.02 | 5.13 | 3.63 | 5.53 | 5.29 | 2.76 | 1.90 | 0.66 |
| | Mean Number of read pairs/group | 1.03 | 1.08 | 1.09 | 1.20 | 1.32 | 1.52 | 1.41 | 2.32 | 3.15 | 7.58 | 11.84 | 32.72 |

| 2 Gbp sequencing data | | | | | | | | | | | | | |
|---|---|---|---|---|---|---|---|---|---|---|---|---|---|
| Library No. | | 1 | 4 | 5 | 6 | 7 | 8 | 9 | 10 | 11 | 12 | 13 | 14 |
| PCR input DNA amount (amol) | | 20000 | 2500 | 1250 | 625 | 313 | 156 | 156 | 78 | 39 | 20 | 10 | 5 |
| Control | Data efficiency (%) | 0.16 | 0.35 | 0,60 | 1.02 | 1.97 | 3.08 | 2.37 | 3.88 | 6.26 | 7.68 | 6.74 | 3.46 |
| | Mean Number of read pairs/group | 1.01 | 1.02 | 1.03 | 1.04 | 1.09 | 1.14 | 1.14 | 1.25 | 1.55 | 2.21 | 4.12 | 8.59 |

### III) Impact of amount of input DNA on overlap rate of sequencing data

### i) 10 Gbp sequencing data

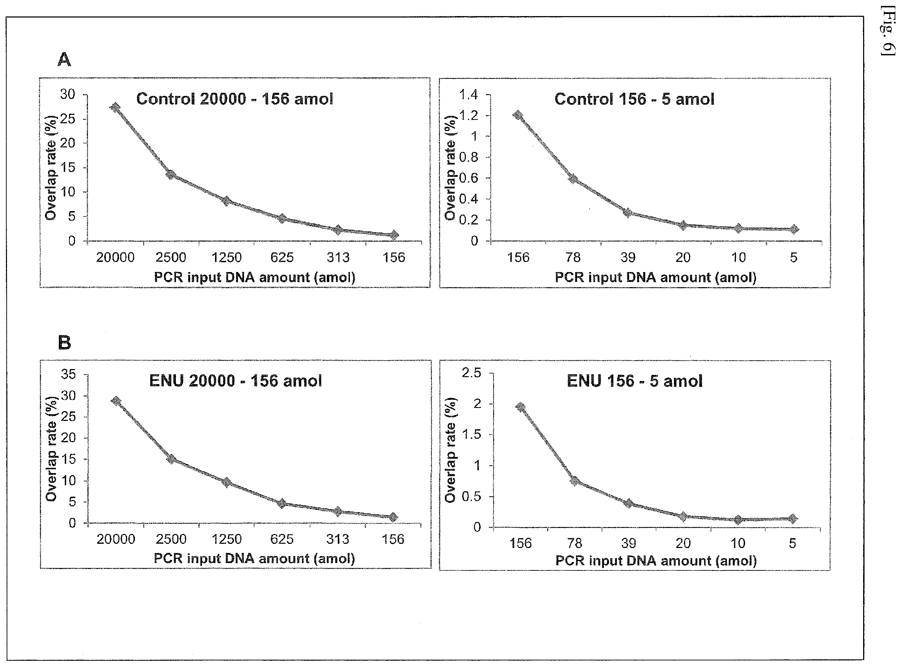

The fraction of groups each relating to one estimated fragment but including read pairs derived from different DNA fragments (overlap rate), which was calculated for the 10 Gbp Fastq file in 7), are shown in Figure 6 and Tables 4 and 5. Figure 6A and Table 4 show data for the DMSO-exposed (control) group, and Figure 6B and Table 5 show data for the ENU-exposed (ENU) group. An overlap rate indicates the fraction of different reads which are derived from different DNA fragments from different cells but misrecognized as reads derived from the same fragment due to their accidentally equivalent sequence information. Because two indexes were used in the present Example, it is inferred that approximately half of events of misrecognizing different DNA fragments as identical DNA fragments were detected. Therefore, a value approximately twice the overlap rate obtained is estimated to be an actual fraction of different DNA fragments the sequences of which happen to contain equivalent sequence information (true overlap rate). For the 10 Gbp sequencing data, the overlap rate decreased as the amount of input DNA in PCR was reduced. The overlap rate with 78 amol (approximately 15.6 amol/Mbp (genome)), the amount of input DNA which provided the highest data efficiency, was 0.59% for the control group, and 0.75% for the ENU group, which suggests that a high data efficiency and a true overlap rate reduced to about 1% can be simultaneously achieved by setting the amount of input DNA to around 78 amol (approximately 15.6 amol/Mbp (genome)).

**[Table 4]**

| Control | | | | | | | | | | | | |
|---|---|---|---|---|---|---|---|---|---|---|---|---|
| Library No. | 1 | 4 | 5 | 6 | 7 | 8 | 9 | 10 | 11 | 12 | 13 | 14 |
| PCR input DNA amount (amol) | 20000 | 2500 | 1250 | 625 | 313 | 156 | 156 | 78 | 39 | 20 | 10 | 5 |
| PCR input / Mbp (genome) | 4000 | 500 | 250 | 125 | 62.6 | 31.3 | 31.3 | 15.6 | 7.8 | 4 | 2 | 1 |
| Total number of groups with >2 read pairs | 789990 | 1565934 | 2264112 | 3589346 | 5762666 | 8080584 | 7391538 | 8968099 | 6788263 | 3952934 | 1817281 | 826902 |
| Number of groups with 1^{st}Index | 310691 | 763538 | 1086879 | 1599464 | 2643429 | 3993483 | 4090814 | 4509930 | 3426909 | 1814305 | 892466 | 399114 |
| Number of groups with 2^{nd} Index | 263240 | 590018 | 991787 | 1826132 | 2990486 | 3989365 | 3211417 | 4404941 | 3343135 | 2132718 | 922665 | 426879 |
| Number of groups with both indexes | 216059 | 212378 | 185446 | 163750 | 128751 | 97736 | 89307 | 53228 | 18219 | 5911 | 2150 | 909 |
| Overlap rate (%) | 27.35 | 13.56 | 8.19 | 4.56 | 2.23 | 1.21 | 1.21 | 0.59 | 0.27 | 0.15 | 0.12 | 0.11 |

**[Table 5]**

| ENU | | | | | | | | | | | | |
|---|---|---|---|---|---|---|---|---|---|---|---|---|
| Library No. | 1 | 4 | 5 | 6 | 7 | 8 | 9 | 10 | 11 | 12 | 13 | 14 |
| PCR input DNA amount (amol) | 20000 | 2500 | 1250 | 625 | 313 | 156 | 156 | 78 | 39 | 20 | 10 | 5 |
| PCR input / Mbp (genome) | 4000 | 500 | 250 | 125 | 62.6 | 31.3 | 31.3 | 15.6 | 7.8 | 4 | 2 | 1 |
| Total number of groups with >2 read pairs | 871423 | 3013638 | 2607096 | 5283784 | 6981568 | 7530681 | 6585067 | 10920911 | 8109174 | 4734379 | 2622344 | 1235504 |
| Number of groups with 1^{st} Index | 315282 | 1490191 | 1192767 | 2821092 | 2936126 | 3354008 | 3452811 | 5486021 | 4155822 | 2294235 | 1290880 | 631743 |
| Number of groups with 2^{nd} Index | 305534 | 1070116 | 1163017 | 2218867 | 3851879 | 4068711 | 3003870 | 5352910 | 3922071 | 2431960 | 1328239 | 602016 |
| Number of groups with both indexes | 250607 | 453331 | 251312 | 243825 | 193563 | 107962 | 128386 | 81980 | 31281 | 8184 | 3225 | 1745 |
| Overlap rate (%) | 28.76 | 15.04 | 9.64 | 4.61 | 2.77 | 1.43 | 1.95 | 0.75 | 0.39 | 0.17 | 0.12 | 0.14 |

### ii) 2 Gbp sequencing data

Table 6 shows overlap rates for the 2 Gbp Fastq file for the control group. It was found that overlap rates tended to be lower to some extent than those for the 10 Gbp sequencing data; however, no significant difference was found.

**[Table 6]**

| Control | | | | | | | | | | | | |
|---|---|---|---|---|---|---|---|---|---|---|---|---|
| Library No. | 1 | 4 | 5 | 6 | 7 | 8 | 9 | 10 | 11 | 12 | 13 | 14 |
| PCR input DNA amount (amol) | 20000 | 2500 | 1250 | 625 | 313 | 156 | 156 | 78 | 39 | 20 | 10 | 5 |
| PCR input / Mbp (genome) | 4000 | 500 | 250 | 125 | 62.6 | 31.3 | 31.3 | 15.6 | 7.8 | 4 | 2 | 1 |
| Total number of groups with >2 read pairs | 71875 | 128267 | 200278 | 324282 | 588306 | 882830 | 775318 | 1180509 | 1748845 | 1987957 | 1522713 | 804308 |
| Number of groups with 1^{st} Index | 29721 | 60245 | 91129 | 150217 | 263525 | 417418 | 339891 | 592836 | 865164 | 996323 | 770114 | 389044 |
| Number of groups with 2^{nd} Index | 27270 | 52691 | 93816 | 159290 | 311138 | 453231 | 424834 | 579061 | 877875 | 988514 | 751552 | 414910 |
| Number of groups with both indexes | 14884 | 15331 | 15333 | 14775 | 13643 | 12181 | 10593 | 8612 | 5806 | 3120 | 1047 | 354 |
| Overlap rate (%) | 20.71 | 11.95 | 7.66 | 4.56 | 2.32 | 1.38 | 1.37 | 0.73 | 0.33 | 0.16 | 0.07 | 0.04 |

### IV) Impact of amount of input DNA on mutation frequency

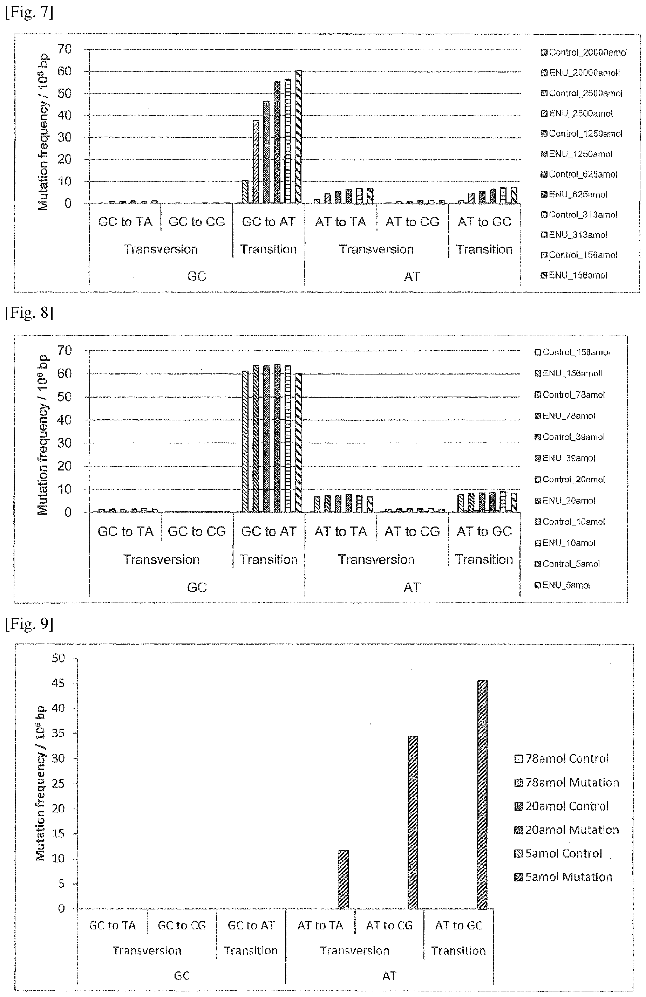

Figures 7 and 8 each show mutation frequencies for mutations detected from the 10 Gbp sequencing data for each library, which were calculated in 8). For the ENU group, the frequency of GC → AT mutation was the highest for each library. This result was consistent with a known result of mutation spectrum analysis for mutations caused by ENU (Matsuda et al. Genes and Environment, 2015, 37: 15-24). For the libraries with the amount of input DNA in PCR of 20,000 to 156 amol (approximately 4,000 to 31.3 amol/Mbp (genome)), it was found that mutation frequencies in the ENU group tended to increase as the amount of input DNA was reduced (Figure 7). For the libraries with 156 to 5 amol (approximately 31.3 to 1 amol/Mbp (genome)), however, the increase of mutation frequencies depending on amount of input DNA was not found (Figure 8). In particular, as the increase of mutation frequencies due to a lowered overlap rate was not found, the impact of overlapping on mutation frequencies was within an experimental error range under conditions with the amount of input DNA in PCR of 78 amol to 5 amol, which provided overlap rates below 1%, and hence it was considered that true mutation frequencies would be provided under conditions close to the above conditions. In view of this, the library with 20 amol (approximately 4 amol/Mbp (genome)), which provided the highest frequency of GC → AT mutation among the above conditions, was used as a reference to calculate relative mutation frequencies of GC → AT mutation for the libraries, which are shown together with overlap rates in Table 7. The results suggested that in the case of the genome of S. typhimurium (genome size: approximately 4.86 Mbp), mutations are detectable with an accuracy of at least 70% or higher if a library is used with the amount of input DNA of about 1,250 amol (approximately 250 amol/Mbp (genome)) or smaller, or with the diversity of DNA equivalent or lower thereto. Meanwhile, mutation frequencies decreased as the amount of input DNA in PCR was larger. From overlap rates for the libraries shown in Table 7, it was inferred that conditions with larger amount of input DNA in PCR provided high overlap rates to result in misrecognizing mutations which should be detected in proper cases as errors, leading to failure in calculation of accurate mutation frequencies.

**[Table 7]**

| Library No. | 1 | 4 | 5 | 6 | 7 | 8 | 9 | 10 | 11 | 12 | 13 | 14 |
|---|---|---|---|---|---|---|---|---|---|---|---|---|
| PCR input DNA amount (amol) | 20000 | 2500 | 1250 | 625 | 313 | 156 | 156 | 78 | 39 | 20 | 10 | 5 |
| PCR input / Mbp (genome) | 4000 | 500 | 250 | 125 | 62.6 | 31.3 | 31.3 | 15.6 | 7.8 | 4 | 2 | 1 |
| GC to AT mutation frequency/10⁶ bp | 10.48 | 37.70 | 46.50 | 55.30 | 56.57 | 60.42 | 61.01 | 63.62 | 63.38 | 63.89 | 63.35 | 60.05 |
| Relative mutation frequency to 20 amol | 16.41 | 59.01 | 72.79 | 86.56 | 88.55 | 94.57 | 95.50 | 99.59 | 99.20 | 100.00 | 99.16 | 94.00 |
| Overlap rate (%) | 28.76 | 15.04 | 9.64 | 4.61 | 2.77 | 1.43 | 1.95 | 0.75 | 0.39 | 0.17 | 0.12 | 0.14 |

### V) Examination on optimum sequencing conditions

Different conditions in sequencing in the present Example are shown in Table 8. Numerical values in columns in Table 8 indicate the following theoretical values, setting values, and measurements.
A. Amount of input DNA in PCR (amol)
B. Amount of input DNA in PCR per 1 Mbp of sample DNA (amol/Mbp (sample DNA))
C. Total number of DNA fragments at initiation of PCR (number of fragments: [6.02 * 10²³ fragments] * A)
D. Number of PCR cycles
E. Amplification rate for each fragment in PCR (C-th power of 2)
F. Total number of fragments after PCR amplification (B * D)
G. Ratio of PCR products each derived from same DNA fragment to E (D/E)
H. Total number of bases sequenced (amount of sequencing data)
I. Number of read pairs in sequencing data
J. Measurement of overlap rate (fraction of different DNA fragments misrecognized as identical DNA fragments)
K. Measurement of number of read pairs per group for estimated fragment
L. Measurement of data efficiency

**[Table 8]**

| A | B | C | D | E | F | G | H | I | J | K | L |
|---|---|---|---|---|---|---|---|---|---|---|---|
| PCR input DNA amount (amol) | PCR input /Mbp (sample DNA) | Initial no. of distinct fragments | PCR cycles | Amplification rate | No. of amplified fragments | Ratio of distinct fragments | No. of base pairs sequenced (Gbp) | No. of read pairs sequenced (M reads) | Overlap rate (%) | Mean Number of read pairs/group | Data efficiency |
| 20000 | 4000 | 1.20E+10 | 7 | 2⁷ | 1.54E+12 | 8.31E-11 | 11.12 | 44 | 27.35 | 1.03 | 0.40 |
| | | | | | | | 2 | 10 | 20.71 | 1.01 | 0.16 |
| 2500 | 500 | 1.51E+09 | 10 | 2¹⁰ | 1.54E+12 | 6.64E-10 | 11.16 | 45 | 13.56 | 1.06 | 0.89 |
| | | | | | | | 2 | 10 | 11.95 | 1.02 | 0.35 |
| 1250 | 250 | 7.53E+08 | 11 | 2¹¹ | 1.54E+12 | 1.33E-09 | 10.61 | 42 | 8.19 | 1.09 | 1.42 |
| | | | | | | | 2 | 10 | 7.66 | 1.03 | 0.60 |
| 625 | 125 | 3.76E+08 | 12 | 2¹² | 1.54E+12 | 2.66E-09 | 10.64 | 43 | 4.56 | 1.16 | 2.32 |
| | | | | | | | 2 | 10 | 4.56 | 1.04 | 1.02 |
| 313 | 62.5 | 1.88E+08 | 13 | 2¹³ | 1.54E+12 | 5.31E-09 | 10.66 | 43 | 2.23 | 1.32 | 3.96 |
| | | | | | | | 2 | 10 | 2.32 | | 1.97 |
| 156 | 31.3 | 9.39E+07 | 14 | 2¹⁴ | 1.54E+12 | 1.06E-08 | 11.44 | 46 | 1.21 | 1.61 | 4.01 |
| | | | | | | | 2 | 10 | 1.37 | 1.14 | 2.37 |
| 78 | 15.6 | 4.70E+07 | 15 | 2¹⁵ | 1.54E+12 | 2.13E-08 | 14.37 | 72 | 0.59 | 2.35 | 4.95* |
| | | | | | | | 2 | 10 | 0.73 | 1.25 | 3.88 |
| 39 | 7.8 | 2.35E+07 | 16 | 2¹⁶ | 1.54E+12 | 4.26E-08 | 14.27 | 71 | 0.27 | 4.12 | 4.17 |
| | | | | | | | 2 | 10 | 0.33 | 1.55 | 6.26 |
| 20 | 4 | 1.20E+07 | 17 | 2¹⁷ | 1.58E+12 | 8.31E-08 | 13.31 | 67 | 0.15 | 7.38 | 2.57 |
| | | | | | | | 2 | 10 | 0.16 | 2.21 | 7.68* |
| 10 | 2 | 6.02E+06 | 18 | 2¹⁸ | 1.58E+12 | 1.66E-07 | 16.80 | 84 | 0.12 | 21,04 | 0.99 |
| | | | | | | | 2 | 10 | 0.07 | 4.12 | 6.74 |
| 5 | 1 | 3.01E+06 | 19 | 2¹⁹ | 1.58E+12 | 3.32E-07 | 13.02 | 65 | 0.11 | 35.28 | 0.56 |
| | | | | | | | 2 | 10 | 0.04 | 8.59 | 3.46 |

| | | | | | | | | | | | |
|---|---|---|---|---|---|---|---|---|---|---|---|
| *: Most efficient condition | | | | | | | | | | | |

### i) Maximization of data efficiency

The data shown in Table 3 revealed that data efficiency in sequencing depended on the amount of sequencing daa used for analysis and amount of input DNA in PCR. In the present Example, no particular limitation was set for the number of read paris included in one group for an estimated fragment in extracting a consensus read sequence considering complementary strands. When at least one set of two read pairs derived from two complementary strands was included in a group, a consensus read sequence considering complementary strands was generated. As a result, it was revealed that optimum data efficiency is achieved when the amount of input DNA is an amount leading to the number of read pairs included in each group for an estimated fragment of approximately 2 on average (Table 3).

### ii) Minimization of overlap rate

The data shown in Tables 4, 5, and 6 revealed that the overlap rate (fraction of different DNA fragments misrecognized as identical fragments) depended on amount of input DNA in PCR and did not depend on the amount of sequencing data used for analysis. Overlap rates are determined from the ratio between (1) the probability that PCR products derived from the same DNA fragment in a library pool are sequenced and (2) the probability that PCR products derived from different DNA fragments and containing equivalent sequence information are sequenced. On the basis of the values in Table 8, if the amount of input DNA in PCR is reduced and the number of PCR cycles is increased, then the fraction of products each derived from the same fragment in the total PCR products increases, which increases the probability of (1) and inversely decreases the probability of (2), and as a result the fraction of misrecognized fragments is lowered, which allows more accurate analysis. As fragments to be sequenced are picked up randomly from PCR products containing highly excessive DNA molecules as shown in E of Table 8, however, the ratio between (1) and (2) in sequencing data becomes comparable to that for PCR products, without depending on data amount. This was inferred to be the cause for the comparable overlap rates in the sequencing for 2 Gbp and 10 Gbp.

### iii) Sequencing conditions for mutation analysis

The accuracy of mutation analysis under the conditions which provided the maximum data efficiency in the present Example (amount of input DNA: 78 amol, amount of sequencing data: 10 Gbp) (Table 7) was determined to be sufficiently high for measurement of frequency of mutation generated by exposing salmonella (genome size: approximately 4.86 Mbp) to a mutagenic substance. Accordingly, the sequencing conditions which provided the maximum data efficiency calculated in i) of V) above were considered to be sufficient conditions for understanding a big picture of genomic mutations introduced by chemicals or the like.

Table 9 shows the relation between data efficiency and amount of input DNA in PCR and amount of sequencing data in the present Example. The amounts of sequencing data per 1 amol of the amount of input DNA in PCR for the libraries with 39, 20, and 10 amol, which provided high data efficiency (> 4%) in analysis for the sequencing for approximately 2 Gbp (10 M read pairs), were 0.26, 0.50, and 1.00 M read pairs (51, 100, and 200 Mbp as the amount of base pairs in read pairs) / amol, respectively. On the other hand, the amounts of the sequencing data per 1 amol of the amount of input DNA in PCR for the libraries with 156 (first), 156 (second), 78, and 30 amol, which provided high data efficiency (> 4%) in analysis for the sequencing for approximately 10 Gbp, were 0.29, 0.41, 0.92, and 1.83 M read pairs (73, 82, 184, and 366 Mbp as the amount of base pairs in read pairs) / amol, respectively. From the results, amount of sequencing data to achieve relatively high data efficiency (> 4%) was calculated to be 0.2 to 2 M (40 to 400 Mbp) read pairs per 1 amol of the amount of input DNA in PCR. The amounts of sequencing data for the libraries with 20 amol for the 2 Gbp sequencing and with 78 amol for the 10 Gbp sequencing, which provided the maximum data efficiency in each case, were 0.50 M and 0.92 M read pairs / amol, respectively, and from the results, amount of sequencing data to maximize data efficiency was calculated to be 0.5 to 1 M (100 to 200 Mbp) read pairs per 1 amol of the amount of input DNA in PCR.

**[Table 9]**

| Relation between amount of sequencing data and amount of input DNA in PCR | | | | | | | | | | | | |
|---|---|---|---|---|---|---|---|---|---|---|---|---|
| Analysis for 2 Gbp data | | | | | | | | | | | | |
| | PCR input DNA amount (amol) | | | | | | | | | | | |
| PCR input (amol) | 20000 | 2500 | 1250 | 625 | 313 | 156 | 156 | 78 | 39 | 20 | 10 | 5 |
| PCR input / Mbp (sample DNA) | 4000 | 500 | 250 | 125 | 62.5 | 31.3 | 31.3 | 15.6 | 7.8 | 4 | 2 | 1 |
| Data efficiency (%) | 0.16 | 0.35 | 0.60 | 1.02 | 1.97 | 3.08 | 2.37 | 3.88 | 6.26 | 7.68 | 6.74 | 3.46 |
| Mean Number of read pair / group | 1.01 | 1.02 | 1.03 | 1.04 | 1.09 | 1.14 | 1.14 | 1.25 | 1.55 | 2.21 | 4.12 | 8.59 |
| reads pair sequenced (M read pair) | 10 | 10 | 10 | 10 | 10 | 10 | 10 | 10 | 10 | 10 | 10 | 10 |
| base pair sequenced (Gbp)* | 2 | 2 | 2 | 2 | 2 | 2 | 2 | 2 | 2 | 2 | 2 | 2 |
| read pair (M reads) / PCR input (amol) | 0.001 | 0.004 | 0.01 | 0.02 | 0.03 | 0.06 | 0.06 | 0.13 | 0.26 | 0.50 | 1.00 | 2.00 |
| base pair (Mbp) / PCR input (amol) | 0.10 | 0.80 | 1.60 | 3.20 | 6.39 | 12.82 | 12.82 | 25.64 | 51.28 | 100.00 | 200.00 | 400.00 |

| | | | | | | | | | | | | |
|---|---|---|---|---|---|---|---|---|---|---|---|---|
| *: Assuming 2 × 100 bp | | | | | | | | | | | | |

| Analysis for 10 Gbp data | | | | | | | | | | | | |
|---|---|---|---|---|---|---|---|---|---|---|---|---|
| | PCR input DNA amount (amol) | | | | | | | | | | | |
| PCR input (amol) | 20000 | 2500 | 1250 | 625 | 313 | 156 | 156 | 78 | 39 | 20 | 10 | 5 |
| PCR input /Mbp (sample DNA) | 4000 | 500 | 250 | 125 | 62.5 | 31.3 | 31.3 | 15.6 | 7.8 | 4 | 2 | 1 |
| Data efficiency (%) | 0.40 | 0.89 | 1.42 | 2.32 | 3.96 | 5.50 | 4.01 | 4.95 | 4.17 | 2.57 | 0.99 | 0.56 |
| Mean Number of read pair / group | 1.03 | 1.06 | 1.09 | 1.16 | 1.32 | 1.60 | 1.61 | 2.35 | 4.12 | 7.38 | 21.04 | 35.28 |
| reads pair sequenced (M read pair) | 44 | 45 | 42 | 43 | 43 | 46 | 64 | 72 | 71 | 67 | 84 | 65 |
| base pair sequenced (Gbp) | 11.12 | 11.15 | 10.61 | 10.64 | 10.66 | 11.44 | 12.87 | 14.37 | 14.27 | 13.31 | 16.80 | 13.02 |
| read pair (M reads) / PCR input (amol) | 0.002 | 0.02 | 0.03 | 0.07 | 0.14 | 0.29 | 0.41 | 0.92 | 1.83 | 3.33 | 8.40 | 13.02 |
| base pair (Mbp) / PCR input (amol) | 0.56 | 4.46 | 8.49 | 17.03 | 34.05 | 73.30 | 82.49 | 184.24 | 366.01 | 665.44 | 1679.67 | 2603.45 |

The examination in IV) above demonstrated that use of a library with the amount of input DNA in PCR of 1,250 amol (approximately 250 amol/Mbp (genome)) or smaller or with the diversity of DNA equivalent or lower thereto allows mutation analysis for salmonella with frequency of misrecognition (true overlap rate) reduced to approximately 20% or lower (Table 7). In view of this, amount of sequence data was determined that allows acquisition of the genome information of salmonella in sequencing with the maximum efficiency for the amount of input DNA in PCR. The optimum amount of sequencing data when the amount of input DNA was 1,250 amol (approximately 250 amol/Mbp (genome)) was calculated to be approximately 625 to 1,250 M (125 to 250 Gbp) read pairs, and amount of sequencing data to provide considerably high efficiency (> 4%) was calculated to be approximately 250 to 2,500 M (50 to 500 Gbp) read pairs. The amount of sequence data (consensus read sequences considering complementary strands) acquired from such amount of sequencing data by using the algorithm in Example 1 is calculated to be 6.25 Gbp to 12.5 Gbp under the conditions for the maximum efficiency, assuming that the data efficiency is approximately 5% (Table 3) and the frequency of misrecognition (true overlap rate) is approximately 20% or lower as in the present Example. If lowering of efficiency is permitted to some extent, for example, in the case that sequencing is performed for 500 Gbp with assuming that the efficiency is > 4% as described above, approximately 20 Gbp of sequence data can be acquired. This is the maximum data amount available for genomic mutation analysis for salmonella based on the present disclosure.

### iv) Estimation of genome size acceptable for mutation analysis

From the finding that the maximum amount of input DNA available for genomic analysis for salmonella was 1,250 amol (approximately 250 amol/Mbp (genome)) (Table 7) as described in IV), the upper limit of amount of input DNA to achieve high data efficiency and low frequency of misrecognition (true overlap rate) was calculated to be 250 amol per 1Mbp of genome. On the other hand, from the finding that mutation frequencies for mutations detected for the ENU group in the present Example were in the order of 1/10⁶ to 1/10⁵ bp, data amount required for mutation analysis was supposed to be at least 10⁶ bp. On the basis of these values, the smallest genome size to which mutation analysis using the method according to the present invention is applicable was estimated. Table 10 shows the maximum value of applicable amount of input DNA in PCR, the optimum amount of sequencing data, and the maximum value of available amount of sequence data (assuming a data efficiency of 5%) for different genome sizes. The result in Table 10 indicates that 10⁶ bp or more of data amount is available from approximately 10 kbp or more of genome, and thus mutation analysis methods based on the present disclosure are applicable to samples with a genome size of approximately 10 kbp or more.

**[Table 10]**

| Size of genome targeted in analysis | Maximum value of applicable amount of input DNA in PCR | Optimum amount of sequencing data | Maximum value of available amount of sequence data |
|---|---|---|---|
| 5 Mbp | 1250 amol | 125 - 250 Gbp | 6.3 -12.5 Gbp |
| 1 Mbp | 250 amol | 25 - 50 Gbp | 1250 - 2500 Mbp |
| 100 kbp | 25 amol | 2.5 - 5 Gbp | 125 - 250 Mbp |
| 10 kbp | 2.5 amol | 250 - 500 Mbp | 12.5 - 25 Mbp |
| 1 kbp | 0.25 amol | 25 - 50 Mbp | 1.25 - 2.5 Mbp |

### Example. 3

### Mutation analysis using synthesized DNA sequence

In the present Example, the efficacy of the sequencing algorithm described in Example 1 and the impact of events of accidental overlapping of fragments derived from different cells on mutation analysis were examined through mutation analysis for a synthesized DNA sequence of 1,000 bp including base-pair substitution mutation.

### 1) Preparation of sample DNA

A random sequence DNA of 1,000 bp (hereinafter, referred to as control DNA) was produced, and base-pair substitution mutation (A:T base pair) was introduced into the control DNA to produce a DNA (hereinafter, referred to as mutated DNA). These were mixed together to prepare sample DNA including three base-pair substitution patterns at A:T base pairs (A:T > G:C, A:T > C:G, and A:T > T:A) with equal predetermined frequencies. Schematic Diagram 6 illustrates the conceptual diagram of the procedure of preparing the sample DNA.

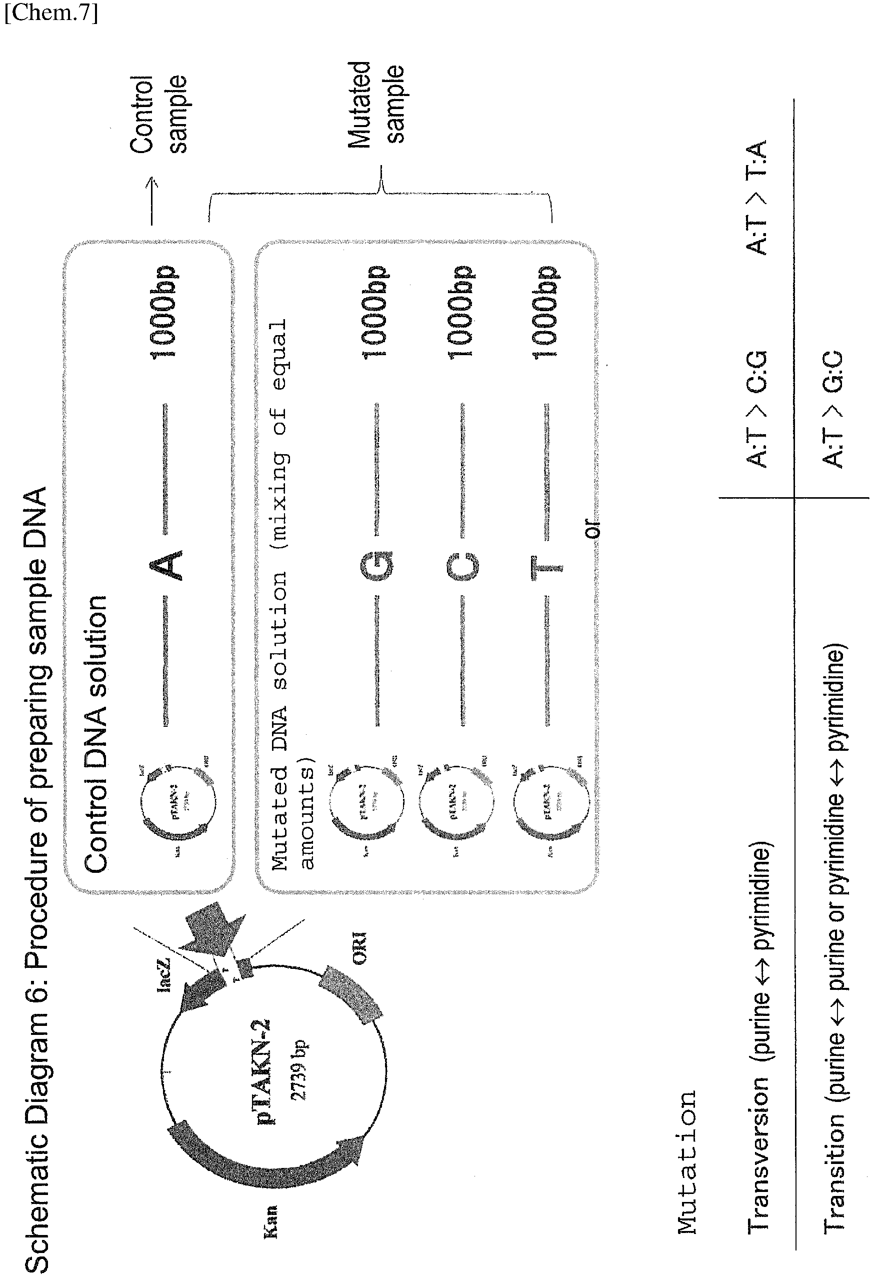

Now, details of the procedure will be described. A DNA of 1,000 bp having a random sequence including approximately 50% of G:C base pairs and approximately 50% of A:T base pairs (control DNA, SEQ ID NO: 1) was synthesized, and incorporated into a pTAKN-2 vector and amplified. Solution of the resulting vector in TE buffer (pH 8.0, produced by Wako Pure Chemical Industries, Ltd.) (10 ng/µL) was prepared (control DNA solution). Mutated DNAs were produced on the basis of the control DNA. In producing the mutated DNAs, the A:T base pair positioned at the center (502nd) of the sequence of the control DNA was substituted with another base pair (A:T > G:C, C:G, or T:A) to produce three sequences. Each of the mutated DNAs was incorporated into a pTAKN-2 vector and amplified, and solution of each resulting vector in TE buffer (10 ng/µL) was prepared. The solutions in equal amounts were mixed together to prepare mutated DNA solution.

The control DNA solution and the mutated DNA solution were mixed together with a ratio shown in Table 11 to prepare a DNA sample (mutated sample) such that the base-pair substitutions were found with equal frequencies and the total mutation frequency was 1/10⁴ bp. As a control sample, a DNA sample not containing the mutated DNA solution (consisting only of the control DNA solution) was prepared. The mutated sample and control sample obtained were subjected to library preparation and sequencing.

**[Table 11]**

| Ratio of mutated DNA solution and total mutation frequency for DNA samples | | |
|---|---|---|
| | Mutated sample | Control sample |
| Total mutation frequency (/bp) | 1/10⁴ | 0 |
| Control DNA solution | 81 µL | 90 µL |
| Mutated DNA solution (base-pair substitution mutation A → G, C, T) | 9 µL | - |
| Ratio of mutated DNA solution | 10 % | 0% |

### 2) Library preparation and sequencing

Libraries were prepared by using the mutated sample and control sample prepared in 1) in the same manner as in Example 2, and sequencing was performed with the resulting libraries. Table 12 shows the amount of input DNA in PCR, index sequences for adapters, and number of PCR cycles in library preparation. Approximately 10 Gbp of sequencing data was acquired from each sample with the corresponding amount of input DNA in PCR.

**[Table 12]**

| Control sample | | | |
|---|---|---|---|
| Library No. | 1 | 2 | 3 |
| DNA concentration (pmol/L) | 3.13 | 0.78 | 0.20 |
| PCR input (amol) | 78 | 20 | 5 |
| 1^{st}Index* | 4 | 6 | 12 |
| 2^{nd} Index* | 14 | 16 | 19 |
| Number of PCR cycles | 15 | 17 | 19 |

| Mutated sample | | | |
|---|---|---|---|
| Library No. | 4 | 5 | 6 |
| DNA concentration (pmol/L) | 3.13 | 0.78 | 0.20 |
| PCR input (amol) | 78 | 20 | 5 |
| 1^{slt}Index* | 3 | 9 | 11 |
| 2^{nd} Index* | 21 | 23 | 27 |
| Number of PCR cycles | 15 | 17 | 19 |

| | | | |
|---|---|---|---|
| *: Identification number of Index | | | |

### 3) Generation of sequence data for mutation analysis, and mutation analysis

Mutation analysis for sequencing data was performed in the same manner as in Example 2. Specifically, the algorithm described in Example 1 was used to generate sequence data containing consensus read sequences considering complementary strands, and base substitutions were detected by using the sequence data through the procedure described in PCT/JP2017/005700.

Edit of sequence data for mutation analysis was performed in accordance with the analysis flow illustrated above in Schematic Diagram 4. First, adapter sequences and bases such as those of low quality were trimmed from raw read sequences to generate read sequence pairs by using the software Cutadapt. For the Fastq files containing the read pairs acquired after trimming, Fastq files derived from the same library were combined into one Fastq file on the basis of index information for adapter sequences. From each of the beginning and end of the resulting Fastq file, 5 * 10⁴ read pairs (10 * 10⁴ read pairs in total) were extracted and the read pairs were combined into one file, and thus a Fastq file for analysis of sequencing data of approximately 20 Mbp was generated.

In analysis by the algorithm described in Example 1 using information on complementary strands, the resulting Fastq file for analysis of data of approximately 20 Mbp was subjected to mapping of read pairs on a reference sequence by using the software Bowtie2 to provide a file in Sam format. The reads of the file in Sam format were sorted by using the software Samtools, and consensus read sequences considering complementary strands were extracted from information on the read pairs mapped on the reference sequence (the sequence of the pTAKN-2 vector into which the control DNA had been inserted) by using programs written in the programming language Python in accordance with the algorithm shown in Example 1. The consensus read sequences considering complementary strands were output as a new Fastq file.

In mutation analysis, the acquired consensus read sequences considering complementary strands were re-mapped on the reference sequence by using the software Bowtie2, and mutated bases were detected by using the software Samtools and programs written in the programming language Python through the procedure described in PCT/JP2017/005700. Specifically, all bases targeted in the analysis in all of the read sequences mapped were classified by the corresponding base (A, T, G, and C) in the reference sequence into four groups by using a program written in Python. Subsequently, the total number of bases was counted and mutated bases as compared with the reference sequence were detected for each group. Such analysis was performed only for bases in read sequences mapped on a region of the 1,000 bp control DNA in the reference sequence.

### 4) Calculation of mutation frequency and increase in mutation frequency

Then, mutation patterns (AT → TA, AT → CG, AT → GC, and GC → TA, GC → CG, GC → AT) were determined for mutated bases detected in each of the mutated sample and the control sample on the basis of the corresponding base in the reference sequence, and the mutation frequency per 10⁶ bps of the target bases was further calculated for each mutation pattern. In the present mutation analysis, base-pair substitution (A:T > C:G mutation) was frequently detected at the 266th A:T base pair, for which no mutation had been introduced, in the region of 1,000 bp as a target of analysis. This was inferred to be due to naturally-occurring mutation in the control DNA (data not shown). Accordingly, read bases mapped on the 266th base were excluded from calculation of mutation frequencies in the present mutation analysis.

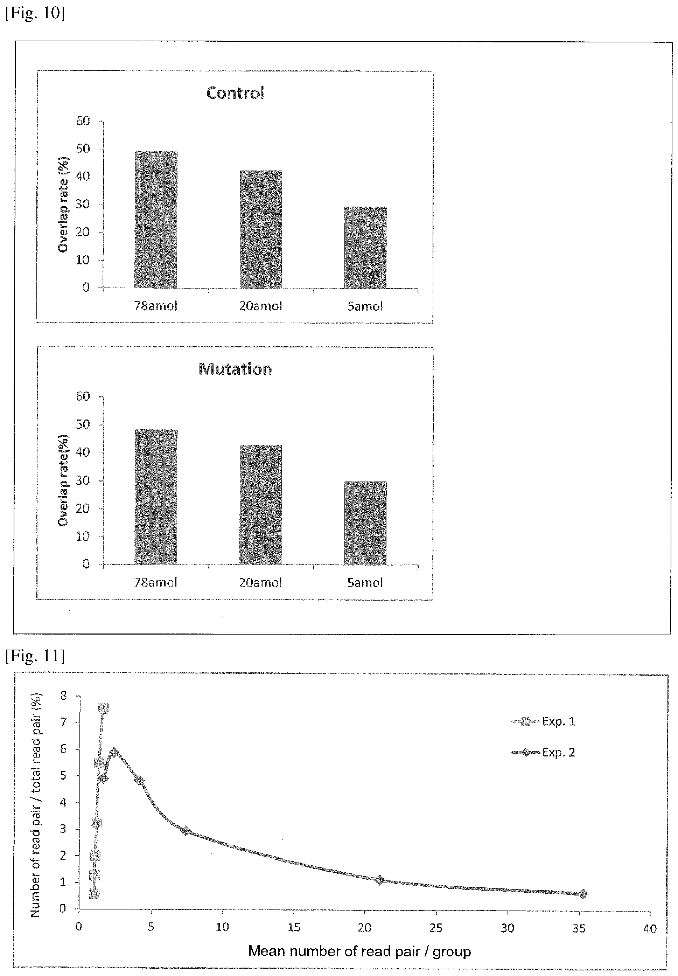

Figure 9 shows mutation frequencies in the samples obtained through analysis by using the algorithm based on the present invention in Example 1 with information on complementary strands. For conditions with the amount of input DNA in PCR of 78 amol and 20 amol, mutation frequency was 0 for all mutation patterns in any of the control sample and the mutated sample. For conditions with the amount of input DNA in PCR of 5 amol, in contrast, base-pair substitution was detected only in the mutated sample with frequencies of approximately 1/10⁵ bps (A:T > T:A) to 5 * 10⁵ bps (A:T > G:C). This probably indicates that a high rate of accidental overlap of different DNA fragments was caused under conditions with 78 amol or those with 20 amol, leading to misrecognizing mutations as errors, while reduction of amount of input DNA in PCR enables detection of mutations. In fact, there was observed a tendency for any of the control sample and the mutated sample that the rate of overlap of different indexes decreased as the amount of input DNA in PCR was reduced from 78 amol to 5 amol (Figure 10). In the design of the present Example, each of the mutation patterns of base-pair substitution is found with a frequency of 1/500 [mutation rate of AT base pairs] bp * 1/3 [number of types of base after being mutated] * 1/10 [dilution rate of mutated DNA solution] = 6.7 * 10⁻⁵ bps. In view of that the rate of overlap of different indexes for the mutated sample under conditions with 5 amol was approximately 30%, the result that the A:T > T:A mutation frequency was as low as approximately 1/7 of the designed value was probably due to the effect of overlap of different fragments.

From these results, it was revealed that the effect of accidental overlap of different fragments can be significant in the case that the target is a small region having the genome size of approximately several kbp, as in the present Example, as compared with the case that a region of larger genome size is targeted. Nevertheless, reduction of amount of input DNA in PCR was found to allow analysis with dramatically lower error frequency through the analysis using information on complementary strands according to the present invention, even in the case of small genome size.

### Example. 4

### Efficiency of generating consensus read sequences considering complementary strands - comparison with Duplex Sequencing

In the present Example, the efficiency of generating consensus read sequences considering complementary strands for 10 Gbp sequence data for the control sample in Example 2 was calculated. For each library as 10 Gbp sequence data for the control sample shown in Table 3 in Example 2, consensus read sequences considering complementary strands were acquired in accordance with the flow illustrated in Schematic Diagram 3. The fraction of the number of consensus read pairs to the total number of read pairs was calculated as the efficiency of generating consensus read sequences considering complementary strands, and plotted against the mean number of read pairs per group for an estimated fragment (Figure 11). In Figure 11, Library Nos. 1 and 4 to 8 for the control sample shown in Table 3 were expressed as Exp. 1, and Library Nos. 9 to 14 as Exp. 2. The results show that the efficiency of generating consensus read sequences considering complementary strands was 7.5% (Exp. 1) and 5.9% (Exp. 2) at maximum.

In the meantime, Duplex Sequencing has been reported to give an efficiency of approximately 1.4% at maximum (Nature protocol, 2014 9(11):2586-2606). The present method provided 4 to 5 times the efficiency thereto. This is probably because while read sequences in groups including three or more read sequences derived from one strand of a fragment of sample DNA are used in generation of consensus sequences in Duplex Sequencing, the present method totally uses groups for estimated fragments, each at least including two read sequences derived from both of two strands, for generating consensus reads considering complementary strands. In generating consensus sequences considering complementary strands in Duplex Sequencing, more reads are used for generating one consensus read than in the present method, which is effective for acquiring more accurate consensus read sequences considering complementary strands; however, Duplex Sequencing is suitable for analysis for relatively short regions, and provides poor efficiency in analysis for wide genome regions. In the present method, in contrast, a relatively small number of reads are used for generating one consensus read, and hence the probability of successfully acquiring consensus reads for a specific genome region is lower but the efficiency of analysis for wide genome regions is enhanced, as described above. Thus, the present method is considered to be a method which is more efficient in performing mutation analysis for the whole genome.

### Example. 5

### Application to mutation analysis using mouse DNA sample

In the present Example, sequencing using information on complementary strands under the optimum conditions found in Example 2 was applied to mutation analysis for genomic DNA from a mouse. Genomic DNA was prepared from a mouse (C57BL/6JJmsSlc-Tg (gpt delta), hereinafter also expressed as a TG mouse) exposed to ENU (ethylnitrosourea, CASRN.759-73-9) as a mutagen, and used as sample DNA. Currently, genetically modified mice or rats transfected with an indicator gene are used for in vivo gene mutation tests using animals. If a mutation analysis method using the present invention is applicable to mice or the like, and as the method does not need use of genetically modified animals, the mutagenicity evaluation can be incorporated in general toxicity tests or the like, leading to reduction of use of experimental animals. Detailed mutation information acquired in the present method is considered to be useful for prediction of carcinogenicity, and thus the present invention may be useful for developing methods of prediction of carcinogenicity without using animals. Animal testing in the present Example were deliberated by the Animal Testing Committee of Research and Development Department of Kao Corporation, and conducted in accordance with "Rules on Animal Testing by Research and Development Department of Kao Corporation".

### 1) Preparation of genomic DNA sample from mice

Male TG mice which were 7 to 9 weeks old at initiation of administration were used. ENU (produced by Toronto Research Chemicals, Inc.) was dissolved in saline to a concentration of 15.0 mg/mL. By using a plastic syringe and a 25 G injection needle, 10 mL of the ENU solution was intraperitoneally administered to the mice once per day (ENU 150 mg/kg/day) for 5 days (ENU group). Saline was administered to a control group. The dose of ENU and the number of individuals for each group are shown in Table 13. Seven days after the final administration, the animals were each euthanized to resect the femur, and genomic DNA was extracted from the bone marrow by using a REcoverEase DNA Isolation Kit (produced by Agilent Technologies) in accordance with the recommended protocol. The double-stranded DNA concentration of the resulting DNA was measured by using a Qubit3.0 Fluorometer (produced by Thermo Fisher Scientific) with a Qubit (TM) dsDNA BR Assay Kit attached thereto.

**[Table 13]**

| Group | Number of animals | Sex | Dose of ENU (mg/kg/day) | ENU concentration of solution (mg/ml) | Administered amount (mL/kg) |
|---|---|---|---|---|---|
| Control | 5 | male | 0 (Saline) | 0 | 10 |
| ENU | 5 | male | 150 | 15.0 | 10 |

### 2) Calculation of gene mutation frequency using gpt gene

DNA was extracted from the bone marrow of each of the mice obtained in 1) by using phenol/chloroform. For the extract DNA, gene mutation frequency was calculated by using the gpt gene introduced into each TG mouse in accordance with OECD Guidelines for the Testing of Chemicals 488 (OECD TG488) (gpt-assay). More specifically, the genomic DNA obtained was packaged in a λ pharge in accordance with Instruction Manual attached to the product of Transpack (Agilent Technologies). The resulting packaging solution was mixed with a suspension of Escherichia coli (YG6020 strain) cultured in advance, and the resultant was cultured and then mixed with a top agar, and the mixture was poured onto an agar medium with chloramphenicol (medium for titer determination) and an agar medium with chloramphenicol and 6-thioguanine (selective medium) to form a layer, and colonies formed were counted. From the number of colonies formed on the selective medium (mutant colonies) and the number of colonies formed on the medium for titer determination (total colonies), mutation frequency (*10⁻⁶) was calculated. For mutant colonies for each DNA sample, some of the colonies were picked up, and confirmation was performed by using the medium for titer determination and the selective medium. The mutation frequency was corrected on the basis of the result of confirmation obtained.

### 3) Preparation of libraries for sequencing

DNA samples from two animals were subjected to experiment for each of the control group and the ENU administration group. Each DNA sample was fragmented into pieces having a length of approximately 350 bp on average by using a DNA Shearing System ME220 (produced by Covaris Inc.) in accordance with the recommended protocol. The fragmented DNA obtained was used for library preparation by using a TruSeq Nano DNA Library Prep Kit (produced by Illumina, Inc., hereinafter abbreviated as TruSeq) in accordance with the recommended protocol. The library preparation was performed in the same manner as in Example 2. Table 14 shows the DNA concentration of each solution, amount of input DNA in PCR, index sequences of adapters, and number of PCR cycles in library preparation.

**[Table 14]**

| Group | Control | | ENU | |
|---|---|---|---|---|
| Library No. | 1 | 2 | 3 | 4 |
| DNA concentration (pmol/L) | 3.13 | 3.13 | 3.13 | 3.13 |
| PCR input (amol) | 78 | 78 | 78 | 78 |
| 1^{st}Index* | 2 | 4 | 10 | 11 |
| 2^{nd} Index* | 13 | 14 | 25 | 27 |
| Number of PCR cycles | 15 | 15 | 15 | 15 |

| | | | | |
|---|---|---|---|---|
| *: Identification number of Index | | | | |

### 4) Sequencing

Each of the libraries prepared in 3) was subjected to sequencing by using a HiSeq 2500 (produced by Illumina, Inc.), where the read length was set to 2 * 100 bp. Sequencing data of approximately 20 Gbp were acquired on average per library.

### 5) Edit of read sequences, extraction of information on complementary strands, and mutation analysis

Edit of raw read sequences acquired through sequencing, extraction of information on complementary strands, and mutation analysis were performed by using the procedure of 5) in Example 2 in accordance with the analysis flow illustrated above in Schematic Diagrams 3 and 4. The genome sequence of C57BL/6J (GCA_000001635.6) was used as a reference sequence for mapping by using the software Bowtie2.

### 6) Calculation of distribution of number of read pairs

From the mapping result generated in 5) for each library, the distribution of the number of read pairs per group for an estimated fragment was determined by using the procedure of 6) in Example 2. For each mouse chromosome, the distribution of the number of read pairs per group and the mean number of read pairs/group were calculated.

### 7) Calculation of overlap rates

By using the procedure of 7) in Example 2, groups including two or more read pairs were extracted from groups for the estimated fragments corresponding to each mouse chromosome, and the overlap rate for each chromosome was calculated for the groups extracted.

### 8) Calculation of mutation frequency

After consensus read sequences considering complementary strands were mapped on the reference sequence in 5), mutated bases were detected by using the procedure of 8) in Example 2. Specifically, for each library, all bases targeted in the analysis in all of the consensus read sequences considering complementary strands mapped on the reference sequence were classified by the corresponding base in the reference sequence into four groups by using a program written in Python, through the procedure described in PCT/JP2017/005700. Subsequently, the total number of bases was counted and mutated bases as compared with the reference sequence were detected for each group. By comparing mutated bases detected and bases in the reference sequence for each of the control group and the ENU group, mutation patterns (AT → TA, AT → CG, AT → GC, and GC → TA, GC → CG, GC → AT) and the mutation frequency for each mutation pattern per 10⁶ bps of each AT base pairs and GC base pairs in the target bases were calculated. Because polymorphism (such as SNP) in the genome sequence possessed by each mouse individual was expected to affect results of mutation analysis, bases in read sequences mapped on a genomic position suspected to include polymorphism with high probability were excluded from targets of the mutation analysis in the present analysis. Specifically, if two or more identical base substitutions were found in read bases mapped on the same position (base) in the reference sequence, the read bases mapped on the position in the reference sequence were excluded from targets of analysis. In addition, bases for which polymorphism had been reported in the reference sequence were excluded from targets of analysis on the basis of mutation information for mice registered in databases. That is, read bases mapped on bases for which polymorphism had been reported in the reference sequence were excluded from targets of analysis. The data on mouse polymorphism were acquired from ensembl [asia.ensembl.org/Mus_musculus/Info/Index].

### 9) Results

### I) Calculation of distribution of number of read pairs

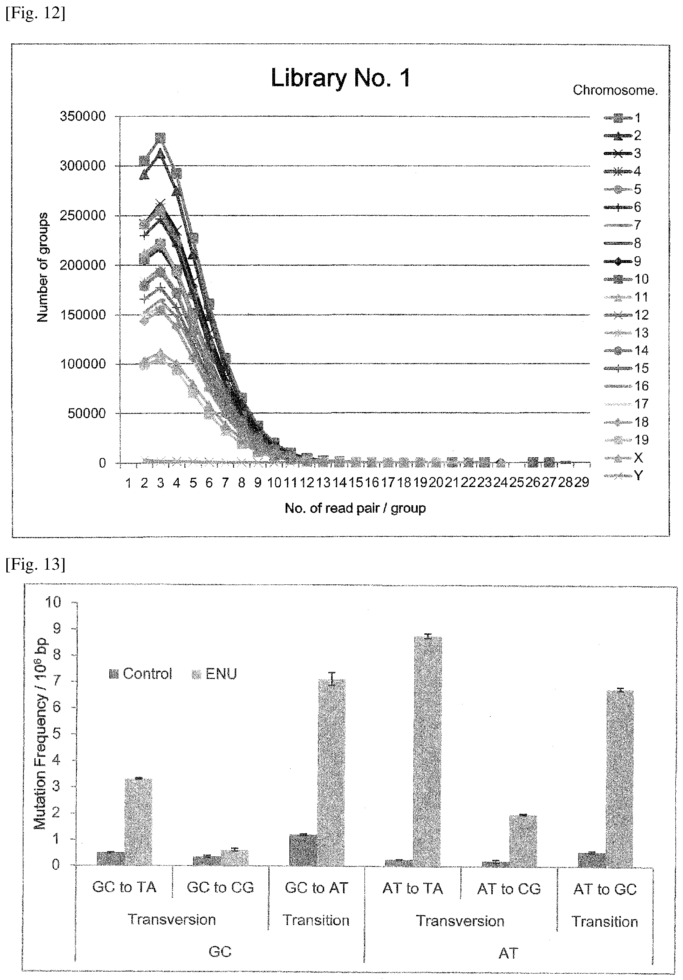

Figure 12 shows the distribution of the number of read pairs per group for an estimated fragment in each mouse chromosome on the basis of the data acquired from Library No. 1. Table 15 shows the mean number of read pairs per group for each chromosome. The values of mean number of read pairs/group for the samples were all within the range of 2 to 4. Hence, it was considered that even in using mouse genomic DNA, high data efficiency is provided through sequencing under the optimum conditions for the present method calculated in Example 2 with respect to amount of input DNA in PCR and amount of sequencing data.

**[Table 15]**

| Mean number of read pairs/group | | | | | | | | | | | |
|---|---|---|---|---|---|---|---|---|---|---|---|
| Library No. | Chromosome | | | | | | | | | | |
| | 1 | 2 | 3 | 4 | 5 | 6 | 7 | 8 | 9 | 10 | 11 |
| 1 | 3.41 | 3.37 | 3.43 | 3.36 | 3.34 | 3.39 | 3.32 | 3.36 | 3.35 | 3.39 | 3.30 |
| 2 | 3.05 | 3.02 | 3.07 | 3.00 | 2.99 | 3.03 | 2.97 | 3.01 | 3.00 | 3.03 | 2.96 |
| 3 | 2.85 | 2.82 | 2.87 | 2.81 | 2.80 | 2.84 | 2.78 | 2.81 | 2.80 | 2.84 | 2.77 |
| 4 | 2.52 | 2.50 | 2.54 | 2.49 | 2.48 | 2.51 | 2.47 | 2.49 | 2.48 | 2.51 | 2.45 |

| Library No. | Chromosome | | | | | | | | | | |
|---|---|---|---|---|---|---|---|---|---|---|---|
| | 12 | 13 | 14 | 15 | 16 | 17 | 18 | 19 | X | Y | |
| 1 | 3.39 | 3.39 | 3.41 | 3.38 | 3.42 | 3.33 | 3.39 | 3.34 | 3.47 | 2.95 | |
| 2 | 3.03 | 3.03 | 3.05 | 3.02 | 3.05 | 2.99 | 3.04 | 2.99 | 3,10 | 2.72 | |
| 3 | 2.84 | 2.84 | 2.85 | 2.83 | 2.85 | 2.79 | 2.84 | 2.80 | 2.90 | 2.44 | |
| 4 | 2.51 | 2.51 | 2.52 | 2.50 | 2.53 | 2.47 | 2.52 | 2.48 | 2.56 | 2.25 | |

### II) Calculation of overlap rates

Table 16 shows overlap rates for the chromosomes examined in 7). The Y chromosome was excluded from targets of the present analysis since it was mapped with less number of reads. Overlap rates were similar among the chromosomes, and the mean among the chromosomes was 0.025% and 0.025% (for Library No. 1 and No. 2) for the control group, and 0.023% and 0.020% (for Library No. 3 and No. 4) for the ENU group. In the examination using the genome sequence of S. typhimurium in Example 2, the overlap rate calculated under the conditions with the amount of input DNA in PCR of 78 amol was 0.59% for the control group, 0.75% for the ENU group, and 0.67% on average. Therefore, it was considered that if the same amount of input DNA in PCR is used, larger size of sample DNA gives a lower overlap rate, allowing analysis with higher accuracy. On the other hand, assuming that the size of mouse genome was approximately 3 Gbp and the size of S. typhimurium genome was approximately 5 Mbp, the overlap rate in genomic analysis for mice as theoretically determined from the results in Example 2 was estimated as [0.67% * 5 Mbp / 3,000 Mbp = 0.0011%]. Thus, the overlap rate actually obtained was about 20 times higher than the theoretical value. In sequencing of genome from mammals such as mice, the size of a region to be actually sequenced is smaller than the genome size because of the presence of heterochromatin and the impact of biases in PCR on library preparation with a next-generation sequencer. This was considered to be a cause for the deviation of the measured value from the theoretical value.

**[Table 16]**

| Overlap rate (%) | | | | | | | | | | | |
|---|---|---|---|---|---|---|---|---|---|---|---|
| Library No. | Chromosome | | | | | | | | | | |
| | 1 | 2 | 3 | 4 | 5 | 6 | 7 | 8 | 9 | 10 | 11 |
| 1 | 0.023 | 0.028 | 0.025 | 0.026 | 0.022 | 0.023 | 0.023 | 0.023 | 0.026 | 0.022 | 0.023 |
| 2 | 0.027 | 0.028 | 0.023 | 0.023 | 0.026 | 0.028 | 0.019 | 0.025 | 0.023 | 0.024 | 0.026 |
| 3 | 0.022 | 0.025 | 0.025 | 0.022 | 0.024 | 0.022 | 0.020 | 0.022 | 0.021 | 0.017 | 0.026 |
| 4 | 0.018 | 0.022 | 0.017 | 0.016 | 0.019 | 0.019 | 0.017 | 0.020 | 0.019 | 0.020 | 0.022 |

| Library No. | Chromosome | | | | | | | | | | |
|---|---|---|---|---|---|---|---|---|---|---|---|
| | 12 | 13 | 14 | 15 | 16 | 17 | 18 | 19 | X | Mean | |
| 1 | 0.024 | 0.028 | 0.022 | 0.025 | 0.021 | 0.030 | 0.024 | 0.026 | 0.026 | 0.025 | |
| 2 | 0.024 | 0.024 | 0.028 | 0.026 | 0.026 | 0.027 | 0.028 | 0.026 | 0.021 | 0.025 | |
| 3 | 0.024 | 0.024 | 0.027 | 0.020 | 0.027 | 0.024 | 0.020 | 0.022 | 0.020 | 0.023 | |
| 4 | 0.018 | 0.023 | 0.021 | 0.018 | 0.020 | 0.020 | 0.021 | 0.020 | 0.019 | 0.020 | |

### III) Results of mutation analysis

Figure 13 shows results of analysis of mutation frequencies for different base-pair substitutions in the control group and the ENU group (n = 2). In the ENU group, the frequencies of A:T > T:A, A:T > G:C, and G:C > A:T were significantly higher than those in the control group, and this result was consistent with existing knowledge on mutation spectra for ENU (Proc Natl Acad Sci USA, 1994, 91(14):6564-6568). It was demonstrated that the present method allows mutation analysis with high sensitivity even for mouse genome.

### IV) Calculation of mutation frequency (gpt-assay)

Table 17 shows results of the gpt-assay calculated in 2) for the control group and the ENU group (control group: n = 5, ENU administration group: n = 4). Significant increase of gene mutation frequency was found for the ENU group, as compared with the control group. It was confirmed that the exposure to ENU introduced mutations into the genome of each TG mouse.

**[Table 17]**

| Group | Dose of ENU (mg/kg/day) | Mean Mutant Frequency | S.D. | N |
|---|---|---|---|---|
| Control | 0 (Saline) | 2.36 | 1.26 | 5 |
| ENU | 150 | 126.33 | 38.64 | 4 |

## Claims

1. A method for sequencing DNA, the method comprising:
(1) preparing fragments of sample DNA;
(2) subjecting the fragments of the sample DNA to PCR to produce two or more amplified fragments for each of the fragments of the sample DNA, and obtaining PCR products containing a plurality of amplified fragments,
wherein the amount of input DNA in the PCR has been adjusted **to 0.002 to 1.7 amol** per 1 Mbp in size of the sample DNA;
(3) sequencing the PCR products to generate one or more read sequences from the plurality of amplified fragments, and acquiring a plurality of read sequences for the plurality of amplified fragments;
(4) collecting, from the plurality of read sequences acquired, read sequences containing sequence information on the same region in the sample DNA into a group to generate one or more groups of read sequences; and
(5) building consensus of sequence information among read sequences included in each of the groups of read sequences.

2. The method of claim 1, wherein each of the one or more groups of read sequences in step (4) include 1.05 to 10 read sequences on average.

3. The method according to claim 1 or 2, wherein the plurality of read sequences acquired in the step (3) includes two or more read sequences generated for each of amplified fragments derived from one and the other of two complementary strands constituting one fragment of the sample DNA.

4. The method according to any one of claims 1 to 3,
wherein the step (4) includes classifying read sequences to be mapped on the same position in a reference sequence into the same group.

5. The method according to claim 3 or 4, wherein the step (5) includes collecting, from each of the groups of read sequences, at least one read sequence derived from each of the two complementary strands constituting one fragment of the sample DNA, and building consensus of sequence information among the collected read sequences.

6. The method according to claim 1, wherein
the plurality of read sequences in the step (3) includes a plurality of read sequence pairs each consisting of read 1 and read 2, wherein
read 1 is a read sequence containing sequence information on reading out of the sequence of one strand of two complementary strands constituting one of the amplified fragments, from the 5'-terminal side to the 3'- terminal side, and
read 2 is a read sequence containing sequence information on reading out of the sequence of the one strand from the 3'-terminal side to the 5'-terminal side;
the step (4) includes collecting, from the read sequence pairs acquired, read sequence pairs containing sequence information on the same region in the sample DNA into a group to generate one or more groups of read sequence pairs; and
the step (5) includes building consensus of sequence information among read sequences included in each of the groups of read sequence pairs.

7. The method according to claim 6, wherein each of the one or more groups of read sequence pairs in step (4) include 1.05 to 10 read sequence pairs on average.

8. The method according to claim 6 or 7, wherein the plurality of read sequence pairs acquired in step (3) includes two or more read sequence pairs generated for each of amplified fragments derived from one and the other of two complementary strands constituting one fragment of the sample DNA.

9. The method according to any one of claims 6 to 8,
wherein the step (4) includes mapping read 1 and read 2 of each of the read sequence pairs on a reference sequence, and classifying read sequence pairs into the same group such that a region between the beginning of read 1 and the beginning of read 2 in the reference sequence is the same thereamong.

10. The method according to any one of claims 8 or 9,
wherein the step (5) includes collecting, from each of the groups of read sequence pairs, at least one read sequence pair derived from each of the two complementary strands constituting one fragment of the sample DNA, and building consensus of sequence information among read sequences included in the collected read sequence pairs.

11. The method according to any one of claims 1 to 5,
wherein 0.02 to 10 * 10⁶ read sequences are acquired in the sequencing per 1 amol of the amount of input DNA in the PCR.

12. The method according to any one of claims 6 to 10,
wherein 0.02 to 10 * 10⁶ read sequence pairs are acquired in the sequencing per 1 amol of the amount of input DNA in the PCR.

13. The method according to any one of claims 1 to 12,
wherein the sample DNA has a size of 10 kbp or more.

14. The method according to any one of claims 1 to 13,
wherein 0.05 to 1,600 * 10⁶ read sequences or read sequence pairs are acquired in the sequencing per 1 Mbp of the sample DNA.

15. The method according to any one of claims 1 to 14,
wherein no label for individually identifying the fragments of the sample DNA is linked to each of the fragments of the sample DNA to be subjected to the PCR.

## Patentansprüche

1. Verfahren zum Sequenzieren von DNA, wobei das Verfahren umfasst:
(1) Herstellen von Fragmenten einer Proben-DNA;
(2) Unterziehen der Fragmente der Proben-DNA einer PCR, um zwei oder mehr amplifizierte Fragmente für jedes der Fragmente der Proben-DNA herzustellen, und Erhalten von PCR-Produkten, die eine Vielzahl von amplifizierten Fragmenten enthalten,
wobei die DNA-Inputmenge bei der PCR auf 0,002 bis 1,7 amol pro 1 Mbp Größe der Proben-DNA eingestellt wurde;
(3) Sequenzieren der PCR-Produkte, um eine oder mehrere Read-Sequenzen aus der Vielzahl von amplifizierten Sequenzen zu erzeugen,
und Erhalten einer Vielzahl von Read-Sequenzen für die Vielzahl von amplifizierten Fragmenten;
(4) Sammeln aus der Vielzahl der erhaltenen Read-Sequenzen von Read-Sequenzen, die Sequenzinformationen zu der gleichen Region in der Proben-DNA enthalten, in einer Gruppe, um eine oder mehrere Gruppen von Read-Sequenzen zu erzeugen; und
(5) Erstellen eines Konsensus von Sequenzinformationen unter den Read-Sequenzen, die in jeder der Gruppen der Read-Sequenzen umfasst sind.

2. Verfahren nach Anspruch 1, wobei jede der einen oder mehreren Gruppen von Read-Sequenzen in Schritt (4) im Durchschnitt 1,05 bis 10 Read-Sequenzen umfasst.

3. Verfahren nach Anspruch 1 oder 2, wobei die Vielzahl der in Schritt (3) erhaltenen Read-Sequenzen zwei oder mehr Read-Sequenzen umfassen, die für jedes der amplifizierten Fragmente erzeugt wurden, die von einem oder dem anderen der zwei komplementären Stränge abgeleitet sind, aus denen ein Fragment der Proben-DNA besteht.

4. Verfahren nach einem der Ansprüche 1 bis 3, wobei Schritt (4) das Klassifizieren der Read-Sequenzen, die an derselben Position mit einer Referenzsequenz gemappt werden sollen, in derselben Gruppe umfasst.

5. Verfahren nach Anspruch 3 oder 4, wobei Schritt (5) das Sammeln aus jeder der Gruppen von Read-Sequenzen mindestens einer Read-Sequenz umfasst, die von jedem der beiden komplementären Stränge abgeleitet ist, aus denen ein Fragment der Proben-DNA besteht, und Erstellen eines Konsensus von Sequenzinformationen unter den gesammelten Read-Sequenzen.

6. Verfahren nach Anspruch 1, wobei
die Vielzahl von Read-Sequenzen in Schritt (3) eine Vielzahl von Read-Sequenzpaaren umfasst, die jeweils aus Read 1 und Read 2 bestehen, wobei
Read 1 eine Read-Sequenz ist, die Sequenzinformationen zum Read-out der Sequenz eines Strangs von den zwei komplementären Strängen enthält, aus denen eines der amplifizierten Fragmente besteht, vom 5'-terminalen Ende zum 3'-terminalen Ende, und
Read 2 eine Read-Sequenz ist, die Sequenzinformationen zum Read-out der Sequenz des einen Strangs vom 3'-terminalen Ende zum 5'-terminalen Ende enthält;
Schritt (4) das Sammeln aus den erhaltenen Read-Sequenzpaaren von Read-Sequenzpaaren, die Sequenzinformationen zu der gleichen Region in der Proben-DNA enthalten, in einer Gruppe umfasst, um eine oder mehrere Gruppen von Read-Sequenzpaaren zu erzeugen; und
Schritt (5) das Erstellen eines Konsensus von Sequenzinformationen unter den Read-Sequenzen umfasst, die in jeder der Gruppen der Read-Sequenzpaare umfasst sind.

7. Verfahren nach Anspruch 6, wobei jede der einen oder mehreren Gruppen von Read-Sequenzpaaren in Schritt (4) im Durchschnitt 1,05 bis 10 Read-Sequenzpaare umfasst.

8. Verfahren nach Anspruch 6 oder 7, wobei die Vielzahl der in Schritt 3 erhaltenen Read-Sequenzpaare zwei oder mehr Read-Sequenzpaare umfasst, die für jedes der amplifizierten Fragmente, das von einem und dem anderen der beiden komplementären Stränge abgeleitet ist, aus denen ein Fragment der Proben-DNA besteht, erzeugt wurden.

9. Verfahren nach einem der Ansprüche 6 bis 8,
wobei Schritt (4) das Mappen von Read 1 und Read 2 jedes der Read-Sequenzpaare mit einer Referenzsequenz und das Klassifizieren der Read-Sequenzpaare in dieselbe Gruppe umfasst, in der Weise, dass innerhalb dieser eine Region zwischen dem Beginn von Read 1 und dem Beginn von Read 2 in der Referenzsequenz jeweils die gleiche ist.

10. Verfahren nach einem der Ansprüche 8 oder 9, wobei
Schritt (5) umfasst: das Sammeln aus jeder der Gruppen von Read-Sequenzpaaren mindestens eines Read-Sequenzpaares, das aus jedem der beiden komplementären Stränge abgeleitet ist, aus denen ein Fragment der Proben-DNA besteht, und Erstellen eines Konsensus von Sequenzinformationen unter den Read-Sequenzen, die in den gewonnenen Read-Sequenzpaaren umfasst sind.

11. Verfahren nach einem der Ansprüche 1 bis 5,
wobei 0,02 bis 10 * 10⁶ Read-Sequenzen beim Sequenzieren pro 1 amol der DNA-Input-Menge in der PCR erhalten werden.

12. Verfahren nach einem der Ansprüche 6 bis 10,
wobei 0,02 bis 10 * 10⁶ Read-Sequenzpaare beim Sequenzieren pro 1 amol der DNA-Input-Menge in der PCR erhalten werden.

13. Verfahren nach einem der Ansprüche 1 bis 12,
wobei die Proben-DNA eine Größe von 10 kbp oder mehr aufweist.

14. Verfahren nach einem der Ansprüche 1 bis 13,
wobei 0,05 bis 1.600 * 10⁶ Read-Sequenzen oder Read-Sequenzpaare bei der Sequenzierung pro 1 Mbp der Proben-DNA erhalten werden.

15. Verfahren nach einem der Ansprüche 1 bis 14,
wobei kein Label zur individuellen Identifizierung der Fragmente der Proben-DNA an jedes der der PCR zu unterziehenden Fragmente der Proben-DNA gebunden ist.

## Revendications

1. Méthode de séquençage de l'ADN, ladite méthode comprenant :
(1) la préparation de fragments d'échantillon d'ADN ;
(2) la soumission des fragments d'échantillon d'ADN à une PCR pour produire deux fragments amplifiés ou plus pour chacun des fragments d'échantillon d'ADN, et l'obtention de produits de PCR contenant une pluralité de fragments amplifiés,
dans laquelle la quantité d'ADN entré dans la PCR a été ajustée à 0,002 à 1,7 amol pour 1 Mbp de taille de l'échantillon d'ADN;
(3) le séquençage des produits de PCR pour générer une ou plusieurs séquences de lecture à partir de la pluralité de fragments amplifiés, et l'acquisition d'une pluralité de séquences de lecture pour la pluralité de fragments amplifiés ;
(4) le rassemblement, à partir de la pluralité de séquences de lecture acquises, des séquences de lecture contenant des informations de séquence sur la même région dans l'échantillon d'ADN en un groupe pour générer un ou plusieurs groupes de séquences de lecture ; et
(5) la formation d'un consensus d'informations de séquence parmi les séquences de lecture incluses dans chacun des groupes de séquences de lecture.

2. Méthode selon la revendication 1, dans laquelle chacun des un ou plusieurs groupes de séquences de lecture à l'étape (4) comprend 1,05 à 10 séquences de lecture en moyenne.

3. Méthode selon la revendication 1 ou 2, dans laquelle la pluralité des séquences de lecture acquises à l'étape (3) comprend deux séquences de lecture ou plus générées pour chacun des fragments amplifiés dérivés de l'un et l'autre des deux brins complémentaires constituant un fragment de l'échantillon d'ADN.

4. Méthode selon l'une quelconque des revendications 1 à 3, dans laquelle l'étape (4) comprend la classification des séquences de lecture à cartographier sur la même position dans une séquence de référence dans le même groupe.

5. Méthode selon la revendication 3 ou 4, dans laquelle l'étape (5) comprend la collecte, à partir de chacun des groupes de séquences de lecture, d'au moins une séquence de lecture dérivée de chacun des deux brins complémentaires constituant un fragment de l'échantillon d'ADN, et la formation d'un consensus d'informations de séquence parmi les séquences de lecture collectées.

6. Méthode selon la revendication 1, dans laquelle la pluralité de séquences de lecture acquises à l'étape (3) comprend une pluralité de paires de séquence de lecture consistant chacune en une lecture 1 et une lecture 2, dans laquelle
la lecture 1 est une séquence de lecture contenant des informations de séquence sur la lecture de la séquence d'un brin des deux brins complémentaires constituant l'un des fragments amplifiés, à partir du côté 5'-terminal vers le côté 3'-terminal, et
la lecture 2 est une séquence de lecture contenant des informations de séquence sur la lecture de la séquence du brin à partir du côté 3'-terminal vers le côté 5'-terminal ;
l'étape (4) comprend le rassemblement, à partir des paires de séquences de lecture acquises, des paires de séquence de lecture contenant des informations de séquence sur la même région dans l'échantillon d'ADN en un groupe pour générer un ou plusieurs groupes de paires de séquences de lecture ; et
l'étape (5) comprend la formation d'un consensus d'informations de séquence parmi les séquences de lecture comprises dans chacun des groupes de paires de séquences de lecture.

7. Méthode selon la revendication 6, dans laquelle chacun des un ou plusieurs groupes de paires de séquences de lecture à l'étape (4) comprend 1,05 à 10 paires de séquences de lecture en moyenne.

8. Méthode selon la revendication 6 ou 7, dans laquelle la pluralité des paires de séquences de lecture acquises à l'étape (3) comprend deux paires de séquences de lecture ou plus générées pour chacun des fragments amplifiés dérivés de l'un et l'autre des deux brins complémentaires constituant un fragment de l'échantillon d'ADN.

9. Méthode selon l'une quelconque des revendications 6 à 8, dans laquelle l'étape (4) comprend la cartographie de la lecture 1 et de la lecture 2 de chacune des paires de séquences de lecture sur une séquence de référence, et la classification des paires de séquences de lecture dans le même groupe de telle sorte qu'une région entre le commencement de la lecture 1 et le commencement de la lecture 2 dans la séquence de référence soit identique pour les deux.

10. Méthode selon l'une quelconque des revendications 8 ou 9, dans laquelle l'étape (5) comprend la collecte, à partir de chacun des groupes de paires de séquences de lecture, d'au moins une paire de séquence de lecture dérivée de chacun des deux brins complémentaires constituant un fragment de l'échantillon d'ADN, et la formation d'un consensus d'informations de séquence parmi les séquences de lecture incluses dans les paires de séquence de lecture collectées.

11. Méthode selon l'une quelconque des revendications 1 à 5, dans laquelle 0,02 à 10 * 10⁶ séquences de lecture sont acquises dans le séquençage pour 1 amol de la quantité d'ADN entré dans la PCR.

12. Méthode selon l'une quelconque des revendications 6 à 10, dans laquelle 0,02 à 10 * 10⁶ paires de séquences de lecture sont acquises dans le séquençage pour 1 amol de la quantité d'ADN entré dans la PCR.

13. Méthode selon l'une quelconque des revendications 1 à 12, dans laquelle l'échantillon d'ADN a une taille de 10 kbp ou plus.

14. Méthode selon l'une quelconque des revendications 1 à 13, dans laquelle 0,05 à 1.600 * 10⁶ séquences de lecture ou paires de séquences de lecture sont acquises dans le séquençage pour 1 Mbp de l'échantillon d'ADN.

15. Méthode selon l'une quelconque des revendications 1 à 14, dans laquelle aucun marqueur pour l'identification individuelle des fragments de l'échantillon d'ADN n'est lié à chacun des fragments de l'échantillon d'ADN devant être soumis à la PCR.
